# EUROPEAN PATENT SPECIFICATION

(11) **EP 1 486 561 B1**
(45) Date of publication and mention of the grant of the patent: **04.12.2013**
(21) Application number: 02790847.4
(22) Date of filing: 24.12.2002
(51) Int. Cl.: C12N 15/09, C12M 1/00, C12M 1/34, C12Q 1/68, G01N 33/53, G01N 37/00

(54) **PROBE MEDIUM**
SONDENMEDIUM
MILIEU DE SONDE

(30) Priority: 26.12.2001 JP 2001394086
(43) Date of publication of application: 15.12.2004
(73) Proprietor: CANON KABUSHIKI KAISHA, Ohta-ku Tokyo 146-8501 (JP)
(72) Inventor: OKADA, Yoshikatsu, CANON KABUSHIKI KAISHA, Ohta-ku, Tokyo 146-8501 (JP); KAMEYAMA, Makoto, CANON KABUSHIKI KAISHA, Ohta-ku, Tokyo 146-8501 (JP); IWATA, Kenichi, CANON KABUSHIKI KAISHA, Ohta-ku, Tokyo 146-8501 (JP)
(74) Representative: TBK
(86) International application number: PCT/JP2002/013440
(87) International publication number: WO 2003/056007

(56) References cited:
- WO-A-99/43688
- JP-A- 2001 178 459
- US-B1- 6 171 797
- PREININGER C. ET AL.: 'Immobilization of oligonucleotides on crosslinked poly (vinyl alcohol) for application in DNA chips' TALANTA vol. 55, no. 5, 13 December 2001, pages 973 - 980, XP002967628

## Description

### TECHNICAL FIELD

The present invention relates to probe medium for immobilizing a probe to a substrate and a method for immobilizing the same. The present invention also relates to a method for detecting a target substance using the substrate with the immobilized probe obtained by immobilizing the probe medium to the substrate.

### BACKGROUND ART

Many methods are known for immobilizing a probe to a substrate. Detailedly, there exist a method for immobilizing a probe by synthesizing the probe on the substrate and a method for immobilizing a probe by attaching a previously prepared probe to the substrate with a pin or stamp. Specifically, a method is known which comprises removing a protecting group from a selected area of the substrate by an activator, biding a monomer having a removable protecting group to the area and repeating these steps thereby synthesizing a polymer having various sequences as disclosed in U.S. Patent No. 5,143,854. There is also known a method which comprises contacting a biologically active substance having a reactivity to carbodiimide group with an immobilizing substance consisting of the substrate and a polymer having a carbodiimide group and carried on the substrate as disclosed in Japanese Patent Application Laid-Open No. 08-23975. Similarly, there is known a method for detecting a biologically active substance by immobilizing the substance to a compound containing carbodiimide group through the carbodiimide group as disclosed in Japanese Patent Application Laid-Open No. 08-334509. Further, as disclosed in Japanese Patent Application Laid-Open No. 2001-178442, there is known a method for immobilizing a DNA fragment to the surface of a solid carrier by contacting a DNA fragment having a thiol end group with a solid carrier in a liquid phase, said solid carrier having a chain molecule which is immobilized on the carrier at one end thereof and has reactive substituent group capable to react with the thiol group to form a covalent bond.

Many methods are also known as a method for detecting a biosubstance. Japanese Patent Application Laid-Open No. 2001-116699 discloses a method for measuring biosubstances such as RNA, CDNA, oligonucleotide easily at a high sensitivity and accuracy eliminating the affects by background noise. As is disclosed in Japanese Patent Application Laid-Open No. 2001-116750, there is also known a method for immobilizing a reactive substance to a substrate by discharging a solution containing the reactive substance from a nozzle at a desired position on the substrate. The substrate is formed of glass or silicon and surface thereof is surface treated for enhancing affinity. The reactive substance is at least one substance selected from the group consisting of DNA fragments, cDNAs, RNAs, enzymes, antigens, antibodies, epitopes, proteins, polynucleotides and peptides.

It is known that a water-soluble polymer material is used for blocking treatment when a probe is immobilized to the substrate. Specifically, it is disclosed in Japanese Patent No. 2794728 as a method comprising immobilizing a sample nucleic acid to a film and dipping the film in a solution containing PVA and/or PVP thereby effecting blocking treatment. As another treating method of a post-treatment of the film on which nucleic acids have been immobilized, blocking treatments using skimmed milk and the like are also known. Specifically, as is disclosed in Japanese Patent Publication No. 06-034756, it comprises immobilizing sample nucleic acids to a film and subjecting the film to blocking treatment with a solution obtained by reacting skim milk and N-succinimidyl-3-(2-pyrridylthio)propionate.

In a substrate with an immobilized DNA probe, it is known to prevent drying of the probe after it is provided on the substrate. Specifically, as is disclosed in Japanese Patent Application Laid-Open No. 2001-178459, it is effected by adding a high boiling solvent to an aqueous solution in which DNA fragments are dissolved or dispersed.

Further, with regard to coating medical devices with a high molecular material (polymer), coating with a polymer and a medical agent is known. Specifically, as is disclosed in International Patent Application Japanese Publication No. 2000-512519, a composition for coating medical devices comprising a biocompatible and biodegradable polymer and a medical agent.

However, among the conventionally used probe medium containing a probe which can specifically bond to a target substance, there has not existed probe medium containing in one media a probe, a site which can bond to the probe and a site which can bond to the substrate. Further, conventional methods for immobilizing a probe which can specifically bond to the target substance to the substrate comprise forming a binding layer on the substrate by coating the substrate with a material containing a site which can bond to the probe or treating the substrate to form a site which can bond to the probe and then providing the substrate with a media containing at least probe to immobilize the probe. Thus, a treatment for forming a site which can bond to the probe on the substrate should be previously effected prior to providing the substrate with the probe medium. Alternatively, a compound having a site which can bond to the probe and the substrate were used to form a material with the immobilized probe and the substrate was previously treated with the material with the immobilized probe to immobilize the probe. Thus, this method requires a treatment of the substrate with a material with the immobilized probe before conducting probe immobilization.

Further, there has not existed probe medium containing a probe which can specifically bond to a target substance, said media containing a probe and a water-soluble polymer material in one media. Although the use of a water-soluble polymer material is known in blocking treatment which is one of the steps constituting a method of detecting a target substance using a substrate with an immobilized probe, there have not existed methods using probe medium containing a water-soluble polymer material or mixing a probe and a water-soluble polymer material at the time of immobilizing the probe onto the substrate. Further, in a conventional substrate with an immobilized probe where the probe which can specifically bond to a target substance has been immobilized onto the substrate, it was known that DNA fragments, a kind of probes, are prevented from being dried after they have been immobilized on the substrate. For this purpose, a high boiling solvent should be added to an aqueous solution in which DNA fragments are dissolved or dispersed.

### SUMMARY OF THE INVENTION

The present disclosure describes probe medium comprising a probe which can specifically bond to a target substance, a site which can bond to the probe and a site which can bond to the substrate.

The present disclosure also describes probe medium comprising a probe which can specifically bond to a target substance, a water-soluble polymer material and a high boiling solvent.

Further, the present disclosure describes a method of immobilizing a probe which comprises providing the probe medium on the substrate by way of the spotting method. The probe on the substrate with the immobilized probe formed by this method of immobilizing a probe provides a DNA array which is a DNA probe. Also provided is a method of detecting a target substance which comprises immobilizing a probe by providing the probe medium on the substrate by way of the spotting method and using the substrate with the immobilized probe thus obtained.

Further, the present disclosure describes probe medium comprising a probe and a substance for immobilizing the probe to the substrate wherein the probe and the substrate are respectively contained in containers and mixed at the time of effecting immobilization to the substrate.

Furthermore, the present disclosure describes probe medium comprising a probe, a water-soluble polymer material and a high boiling solvent which are respectively contained in containers and mixed at the time of effecting immobilization to the substrate.

### DESCRIPTION OF THE PREFERRED EMBODIMENTS

### Embodiments of the invention

The probe medium and the method of immobilizing a probe on the substrate as the first embodiment of the present invention comprise a probe, a substance having a site which can bond to the probe and a substance having a site which can bond to the substrate. The probe medium and the method of immobilizing a probe on the substrate as the second embodiment of the present invention comprise a probe, a water-soluble polymer material and a high boiling solvent.

Examples of probe include a single-stranded nucleic acid probe, single-stranded DNA probe, single-stranded RNA probe and single-stranded PNA probe. The amount of the probe present in the media is preferably adjusted to, for example, 2 mer to 500 mer, particularly 2 mer to 80 mer of the nucleic acid probe and to the level of 0.05 to 500 µmol/l, particularly 0.5 to 50 µmol/l, taking into consideration the stability of nucleic acid probe used as probe medium. These probes may be used singly but preferably they are modified with linker at the end thereof. Examples of linker modification include biotination, modification by a functional group, and the like. Functional groups usable for this purpose include amino (NH₂) group, carbonyl (COOH) group, mercapto (SH) group and hydroxyl (OH) group. Preferred functional group is amino group taking into consideration the readiness of synthesizing probe functional group and mercapto group taking into consideration the reactivity of the probe functional group.

The site which can bond to the probe is a site which can bond to the linker at the end of the probe. It can bond to the linker of the probe end in various ways and preferably through a chemical bond. The site which can bond to the probe linker may include functional groups. The amount of the site which can bond to the probe in the media is not limited and may vary depending on the type of the site which can bond to the probe, but it is preferably adjusted to 100 to 100000 equivalent taking into consideration the binding property to the probe.

The site which can bond to the substrate is not limited but preferably a site which can firmly bind to the substrate. The site may be a functional group and preferably a group which forms a chemical bond with the substrate. The functional groups to bind to the substrate include silanol (SiOH) group Particularly the functional groups which can bond to the substrate is preferably silanol group taking into consideration the readiness of immobilization onto the substrate but when the substrate contains amino group or carbonyl group, functional groups reactive to that one of these are preferable. Silanol group may be formed by hydrolysis of an alkoxylsilyl group. The probe and the substance which immobilizes the probe to the substrate can react with and bind to each other in the media, preferably through a chemical reaction between the functional groups to form a covalent bond.

The probe, site which can bond to the probe and the site which can bond to the substrate can be used in various combinations but specific examples thereof include a combination with silanol group as a functional group at the site which can bond to the substrate. In addition to this, they include silanol group as a functional group at the site which can bond to the substrate. They preferably react in a media by mixing or may be those which are subjected to a treatment under a predetermined condition for proceeding the reaction.

Specific examples of compounds containing a functional group at the site which can bond to the probe and a functional group at the site which can bond to the substrate in one compound in which the latter is silanol group include a silane coupling agent. Silanol group is formed by hydrolysis of the alkoxysilyl group in a silane coupling agent. The other functional group in the silane coupling agent may be epoxy group, isocyanate group, mercapto group and chloropropyl group as listed above, and corresponding silane coupling agents are epoxysilane, isocyanatesilane, mercaptosilane and chloropropylsilane respectively. These silane coupling agents may be hydrolyzed if necessary, and then mixed with a probe.

Specific examples of compounds containing a functional group at the site which can bond to the substrate and a functional group at the site which can bond to the substrate in different compounds in which the former is maleimide group and the latter is silanol group include a combination of a bifucntional agent and a silane coupling agent. Specific examples of bifunctional agents include N- (4-maleimidocaproyloxy) succinimide, N-(6-maleimidocaproyloxy)succinimide, N- (8-maleimidocaproyloxy)succinimide and N-(11-maleimidocaproyloxy)succinimide. When it is preferred that these substances are water-soluble to be handled as probe medium, water-soluble sodium salts derived from these substances, i.e. N-(4-maleimidocaproyloxy)sulfosuccinimide, N-(6-maleimidocaproyloxy)sulfosuccinimide, N-(8-maleimidocaproyloxy)sulfosuccinimide, N-(11-maleimidocaproyloxy) sulfosuccinimide may be used. As a silane coupling agent, the silanol group may be formed from the hydrolysis of an alkoxysilane group. There are various types of silane coupling agent which react with a bifunctional agent and those containing amino group are preferred. These silane coupling agents may be hydrolyzed if necessary, and then mixed with a probe.

Examples of water-soluble polymer material include polyvinyl alcohol (PVA), polyvinylpyrrolidone (PVP), paogen, carboxymethylcellulose(CMC), hydroxyethylcellulose(HEC), dextran and pullulane. Among these, polyvinyl alcohol (PVA) and polyvinylpyrrolidone (PVP) are preferable because they are commonly used material and stable to the probes. In case of commonly used polyvinyl alcohol (PVA), it is preferable to adjust the polymerization degree and saponification number according to need. Solubility of the polyvinyl alcohol in to the probe medium, viscosity of the probe medium and the spot strength of the spots formed by spotting the probe medium on the substrate, hygroscopic property thereof can be adjusted by adjusting the polymerization degree and saponification number. Specifically, the polymerization degree and saponification number are adjusted to attain a suitable probe stability as long as they may not cause troubles in providing the probe medium on the substrate. In addition, spot form and hygroscopic properties etc. of the substrate with the immobilized probe, which is prepared by providing the probe medium on the substrate, can be adjusted to respective suitable states. However, when the polymerization degree of polyvinyl alcohol is too high, solubility of polyvinyl alcohol into the probe medium decreases while viscosity thereof increases, and therefore, the probe medium becomes difficult to be stably provided on the substrate. When the saponification number of polyvinyl alcohol is too high, solubility of polyvinyl alcohol into the probe medium decrease while viscosity thereof increases, and therefore, the probe medium becomes difficult to be provided on the substrate. To avoid these problems, the concentration of polyvinyl alcohol may be lowered but the decreased concentration also lowers probe stability, spot form properties and hygroscopic property, and therefore suitable selection of polyvinyl alcohol and adjustment of concentration are required. The concentration of polyvinyl alcohol in the probe medium is preferably adjusted to 0.001 to 1 wt%. It is particularly adjusted to 0.01 to 0.2 wt%. When the concentration of polyvinyl alcohol in the probe medium falls within this range, it is preferred to decrease the polymerization degree of polyvinyl alcohol and increase the saponification number thereof. Preferred polymerization and saponification numbers of polyvinyl alcohol are, for example, polymerization degree of 200 to 1700 and saponification numbers of 87 to 99 mol%

These polyvinyl alcohols may be previously dissolved and then mixed with the probe medium. Organic solvents such as ethanol and dimethylsulfoxide (DMSO) may be used for dissolving the polyvinyl alcohols, but water and ethanol are preferred taking into consideration of the solubility. Polyvinyl alcohol may be mixed with water and then warmed to rapidly dissolve undissolved contents. Preferably, it is mixed with the probe medium after filtering dissolved polyvinyl alcohol to remove the undissolved contents. The thus prepared polyvinyl alcohol is adjusted to the range of pH 6.0 to pH 9.0, particularly pH 7.0 to pH 8.0 taking into consideration the probe stability when mixed with the probe.

The media of the probe medium comprising a probe and a substance which immobilizes the probe on the substrate is preferably those containing water. Ingredients contained in the media other than water include ethylene glycol, thiodiglycol, glycerine, isopropyl alcohol, ethanol and the like. Specific example of the media is a media comprising 70 to 95 wt% of water, 0.1 to 3 % of thiodiglycol, 5 to 30 wt% of isopropyl alcohol. More preferably media comprises 80 to 90 wt% of water, 1 to 2.5 % of thiodiglycol, 10 to 20 wt% of isopropyl alcohol.

Examples of high boiling solvent include ethylene glycol, propylene glycol, triethylene glycol, diethylene glycol, dipropylene glycol and thiodiglycol. Among these, thiodiglycol and dipropylene glycol are preferable because they are commonly used materials and suitable as spotting media. Glycol based high boiling point organic solvents are highly viscous solvents and therefore, it is difficult to meter these solvents for mixing. Accordingly, it is preferred that they are previously dissolved in a low viscosity solvent such as isopropyl alcohol. Alcohol solvents other than isopropyl alcohol, water and the like may be also used as a low viscosity solvent to be mixed with for this purpose. Addition of the high boiling solvent is preferably adjusted so that the spot form property, hygroscopic property, drying property, dried conditions and so forth are suitable when the probe medium is provided on the substrate.

Specific example of the media comprises 70 to 95 wt% of water, 0.1 to 3 wt% of thiodiglycol, 5 to 30 wt% of isopropyl alcohol, 0.001 to 1 wt% of polyvinyl alcohol. More preferable media comprises 80 to 90 wt% of water, 1 to 2.5 wt% of thiodiglycol, 10 to 20 wt% of isopropyl alcohol, 0.01 to 0.2 wt% of polyvinyl alcohol.

Further, the probe medium may comprise a probe immobilizing substance which helps the probe to be efficiently immobilized to the substrate. Examples of probe immobilizing substance include primer, coupling agent, sealant, surface treatment agent, surface modification agent, a substance in which a coupling agent and a bridging agent are bound to each other, a substance in which a coupling agent and a bifunctional agent are bound to each other. The amount of the substance is preferably adjusted to, for example, 0.05 to 50000 µmol/l, particularly 10 to 500 µmol/l particularly in the media taking into consideration immobilizing properties of the probe onto the substrate. These substances may contain a functional group which bonds to a probe or a functional group of the probe and examples thereof include epoxy (CHOCH₂) group, amino (NH₂) group, mercapto (SH) group, maleimide group, chloropropyl (C₃H₆Cl) group, isocyanate (NCO) group. Particularly the functional groups of the probe are preferably amino (NH₂) group, carbonyl (COOH) group, mercapto (SH) group, hydroxyl (OH) group, and the functional groups which can bond to the probe are preferably epoxy group, maleimide group and chloropropyl group taking into consideration the binding to the probe. These substances to immobilize the probe to the substrate may be substances which have a functional group to be immobilized to the substrate, and examples of the functional group include alkoxysilyl (SiOR) group, silanol (SiOH) group and alkoxy (OR) group. Particularly alkoxysilyl group and silanol group are preferred taking the readiness of immobilization onto the substrate. Silanol group may be formed by hydrolysis of an alkylsilyl group. The probe and the substance which immobilizes the probe onto the substrate can bond to each other in the media through a reaction, preferably through a chemical reaction between the functional groups to form a covalent bond.

In order to mix the probe medium with the water-soluble polymer material, a solution of the water-soluble polymer material of a predetermined concentration in which the water-soluble polymer material is completely dissolved is previously prepared. The thus prepared solution of the water-soluble polymer material is weighed and mixed so that the concentration thereof may be a predetermined concentration. Specifically such a solution of the water-soluble polymer material may be prepared by admixing polyvinyl alcohol powder with pure water to the concentration of 0-5 to 5 wt%. The mixed solution is warmed in order to dissolve the polyvinyl alcohol and then filtered to remove undissolved contents and impurities. Thus the aqueous solution of the water-soluble polymer is prepared. The aqueous solution of the water-soluble polymer is preferably mixed with the probe medium so that the concentration thereof in the probe medium may fall within 0.01 to 0.2 wt%.

The probe medium can be added in various procedures. The first method comprises previously mixing a probe with a substance which has a functional group at the site which can bond to the probe and a functional group at the site which can bond to the substrate, then adding thereto a predetermined amount of media, i.e. solvent such as water or an organic solvent, and mixing the solution. If necessary, the temperature of the mixture is adjusted and after a predetermined time the addition of and mixing with the media may be repeated. At last, either one of a solution not added or added in a less amount is added so that the solution may reach to the predetermined composition, thereby preparing the probe medium. At the step where the probe and the substance are mixed, mixing may start the reaction and therefore, it is preferable that the temperature, time, pH, etc. at the mixing step is adjusted according to need. In the case that the reaction starts by mixing the probe and the substance, they may be previously mixed, which facilitates reaction between the probe and the substance to proceed sufficiently. Further, adjusting the mixing will facilitate the probe and the substance to be bound sufficiently, which is also preferable. When a solution is added or mixed to the mixture media comprising the probe and the substance, each component of a part or whole of the solution such as water and an organic solvent may be sequentially added dropwise, mixed and adjusted, or alternatively components of a part or whole of the solution such as water and an organic solvent may be previously mixed and the mixture may be added dropwise, mixed and adjusted. In the case that the probe and the substance react with each other and the addition of the solvent further promote the reaction, it is preferred that the order, interval and the condition when the addition is effected are adjusted for each of the media component to be added. The second method, which can be adopted only when the probe, the functional group at the site which can bond to the probe and the functional group at the site which can bond to the substrate are contained in different substances, comprises mixing the substance having a functional group at the site which can bond to the probe and either one of the probe or the substance having a functional group at the site which can bond to the substrate with a solution comprising a part or whole of the media such as water and an organic solvent, and then adding and mixing the other one. This method is preferable in the case that the probe or the substance are difficult to be mixed with or dissolved into the solvent because the mixing and dissolving conditions can be adjusted, and also in the case that mixing them all simultaneously will give rise to some effects other than the desired reaction. Particularly, it is preferable in the case that the substance is a silane coupling agent and the functional group which can bond to the probe is stable when mixed with and/or dissolved into the solution, since it enables to form a sufficient amount of silanol group by previously mixing and dissolving the substance. The third method comprises weighing the probe, the substance having a functional group at the site which can bond to the probe and the functional group at the site which can bond to the substrate, and a part or whole of the components constituting the solution respectively, placing them all in a container and mixing them at a time. This method can be adopted in the case that the properties of the probe medium will not change regardless of the order of adding the probe, substance and solution. This is a preferable method because the mixing procedure is simplified.

The procedure of adding a water-soluble polymer material to the probe medium is not limited but it is preferably conducted at the last step after the other media and the probe are mixed.

The method of adding and mixing a high boiling solvent to the probe medium is preferably conducted by previously weighing the high boiling solvent so that the concentration thereof may reach the predetermined value and then adding to and mixing with the probe medium.

The procedure of adding and mixing the high boiling solvent to the probe medium is not limited but it is preferably conducted at the last step after the other media and the probe are mixed.

The probe medium thus obtained is preferably used for detecting the target substance.

A substrate with an immobilized probe can be prepared by spotting the probe medium thus obtained onto the substrate. The substrate on which the probe is immobilized is not limited but glass or quartz substrate material is preferable taking into consideration the facility of detection and availability as commonly used material. Specifically preferred is a slide glass substrate having a size of 1 inch × 3 inch, which is preferably formed of alkali-free slide glass substrate which does note contain alkali ingredients or quartz glass material taking into consideration the immobilization properties of the probe. The shape of the substrate is not limited, for example to the form of a substrate, regardless of the method of detecting the target substance but it is preferably in the form of substrate condition because it can be commonly used and does not depend upon the detection method and the apparatus. Further, it is desirable that the substrate has a highly smooth surface.

The substrate on which the probe is immobilized has preferably a clean surface for uniformly and surely immobilizing the probes on the substrate. It is desired that the substrate is washed before immobilizing the probes for securing a sufficiently clean surface. Various methods of cleaning the surface are known including cleansing with water, cleansing with an agent solution, cleansing with plasma, cleansing with UV ozones. Cleansing with an agent solution is suitable since it can be carried out easily and uniformly. For example, one usable method comprises washing the substrate surface with a sodium hydroxide aqueous solution of a predetermined concentration and removing dirt attached on the substrate. Specifically, a 1 mol/l sodium hydroxide aqueous solution warmed to about 50°C is prepared and the substrate surface is wiped in the aqueous solution or brushed while being applied with a shower of the aqueous solution to surely remove the dirt attached on the substrate. After the dirt was removed, remaining sodium hydroxide is sufficiently washed out with water. And lastly water is removed by, for example, N₂ blow. Thus a substrate on which probe medium can be uniformly and surely immobilized can be obtained.

Some methods are known for spotting the probe medium on the substrate. Specifically, there are known a pin method, ink-jet method, and pin and ring method. Among these, the ink-jet method is a preferable spotting method since it enables high-density and accurate spotting.

Ingredients contained in the probe medium is not limited in conducting spotting by way of ink-jet method as long the probe and the substance which immobilize the probe on the substrate in the probe medium are not substantially affected by discharging the probe medium from the ink-jet head and the probe medium satisfies the condition on media composition to be normally dischargeable onto the substrate using the ink-jet head. For example, when the ink-jet head is a bubble ink-jet head having a mechanism of providing the media with thermal energy to achieve discharging, a liquid containing glycerine, thiodiglycol, isopropyl alcohol is preferred as a component of the probe medium. More specifically, a liquid containing 5 to 10 wt% of glycerine, 5 to 10 wt% of thiodiglycol is preferred as probe medium. When the ink-jet head is a piezo jet head which discharges the media using piezoelectric element, a liquid containing ethylene glycol and isopropyl alcohol is preferred as a component of the probe medium. More specifically, a liquid containing 5 to 10 wt% of ethylene glycol and 0.5 to 2 wt% of isopropyl alcohol is preferred as probe medium.

When the thus obtained probe medium is discharged from the ink-jet head to adhere the media onto the substrate, the form of the spot is circle and will keep to the area as discharged. Accordingly, even in the case that high-density spotting of the probe medium is conducted, links between the adjacent spots is effectively suppressed. Further, the probe medium containing a water-soluble polymer and a high boiling solvent enables the probe to be maintained intact and the target substance Lo be detected even in dry spot condition in which the probe medium is dried after spotted. Further, spot state can be easily observed after dried and can be confirmed that the spots are successfully formed in spotting. In the meantime, properties of the probe medium of the present invention are not limited to those described above.

The probe and the substrate preferably bind to each other through a reaction between the probe and the substrate. The probe is firmly immobilized to the substrate through such a reaction. Accordingly, the probe is immobilized to the substrate and probe spots can be formed on the predetermined positions. A water-soluble polymer material does not adversely affect the immobilization of the probe on the substrate and addition of the water-soluble polymer material in the probe medium provides an effect of delaying the spot drying which occurs after spotting and thereby allowing the reaction to sufficiently proceed.

In order to immobilize the probe contained in the probe medium which is provided on the substrate to the predetermined position, and surely prevent contamination from the probe contained in the adjacent spot and to immobilize the probe firmly to the substrate, there is, as an effective means, a method using a substance which bonds to the site which can bond to the substrate as well as to the substrate through a chemical effect. Such a binding by chemical effect includes various ones and for example, covalent bond, ionic bond and the like. Covalent bond is preferred in order to firmly immobilize the probe to the substrate.

Examples of preferred combination include a combination of silanol (SiOH) group on the substance side and hydroxyl (OH) group on the substrate side. Silanol group of the substrate contained in the probe medium, when provided on the substrate by spotting, reacts with the hydroxyl group on the substrate to immobilize the substance. Specific examples of preferred substrate include glass material and quartz substrate material which have hydroxyl groups on the substrate.

It is also preferred that the probe and the site which can bond to the probe are bonded to each other through a chemical effect. Examples of bonding as a chemical effect include covalent bond and ionic bond. Covalent bond is preferred so as to immobilize the probe firmly to the substrate. As a result, probes are immobilized on the substrate and probe spot can be formed in designed positions. Particularly, probe medium was prepared which comprises a probe having amino functional group and a substance having an epoxy group at the site which can bond to the probe and a silanol group at the site which can bond to the substrate and a probe solution mixed with glycerine, thiodiglycol and isopropyl alcohol was prepared. When this solution is spotted by ink-jet head onto the substrate having hydroxyl groups, the probe solution forms spots of a stable size on the substrate. Thus the probes can be immobilized at designed positions. Another probe medium was also prepared which comprises a probe having amino functional group and a substance having an isocyanate group at the site which can bond to the probe and a silanol group at the site which can bond to the substrate and a probe solution mixed with glycerine, thiodiglycol, isopropyl alcohol and the like was prepared. When this solution is spotted by ink-jet head onto the substrate having hydroxyl groups, the probe solution forms spots of a stable size on the substrate. Thus the probes can be immobilized at designed positions.

It is preferable to having added polyvinyl alcohol (PVA), a water-soluble polymer material, to the probe medium. More preferably, it is desired that it has been completely dissolved. Addition of a water-soluble polymer material to the probe medium facilitates observation of the spotting conditions on the substrate and delays drying of the probe medium on the spot after spotted, thereby reacting the probe medium and the substrate more surely and enabling secure immobilization. Further, it facilitates the probe medium spots to detect the target substance more effectively and stably even in the case the probe medium spots formed by spotting are dried taking into consideration the stored condition of the substrate with the immobilized probe prepared by spotting.

For example, probe medium containing a probe of 20 mer bases was adjusted so that the concentration of probe is 8 µmol/l. Probe medium was discharged using a bubble jet printer (Trade name: BJF850; a product of Canon Inc., converted so that it may be capable to print on a flat plate) with the distance between the solid phase and the nozzle of bubble jet head being set to about 1.2 to 1.5mm, the probe medium discharged from the nozzle (discharge amount: about 4 picoliter) can form a spot having a diameter of about 50 to 80 µm on the substrate and spots derived from the splash when the liquid impacted onto the solid phase surface (hereinafter referred to as "satellite spots") were not observed at all by visual observation using a magnifying glass.

The substrate with the immobilized probe (formed by immobilizing probe medium which does not contain a water-soluble polymer material) was analyzed with a microscopic Fourier transformation infrared spectrometer (microscopic FT-IR). Si-O bond peaks from the substrate was detected and no other peaks were substantially recognized on unspotted sites while a broad peak of the substrate was able to be observed and a peak of -CH₂- bonding of the substance which immobilize the probe contained in the probe medium was also detected although the peak value was very low.

The substrate with the immobilized probe was analyzed by thermal desorption spectrometry (TDS) method. Before analyzing, substrate pieces (7 mm × 7 mm) containing the spotted site and substrate pieces (7 mm × 7 mm) consisted of the unspotted marginal area of the substrate were cut out from the substrate and compared. The temperature was set from 25 to 500°C and the programming rate (temperature elevating rate) to 5°C/min. The both substrates were compared in the mass of eliminated substances, and as a result, many mass peaks attributable to the probe and the substance immobilizing the probe on the substrate such as CH₄ and CH₃OH, etc. were detected over 150°C on the spotted site. It can be confirmed from these facts that probe medium were spotted only on the predetermined spot sites and the area other than the spotted sites were not provided with probe medium or substances contained in the media.

The substrate with the immobilized probe was analyzed with a time of flight - secondary ion mass spectrometer (TOF-SIMS). In analyzing the sites, surface scan was conducted in the vicinity of the spot formed site and compared. As a result, PO2⁻ and P03- ions derived from DNA were detected on spotted area. On the other hand, PO₂⁻ and PO₃⁻ ions derived from DNA were not detected on unspotted area as well as between the spots, and also no secondary ions derived from the functional groups at the site which can bond to the probe.

In the present invention, for the purpose of improving the detection accuracy (SN ratio) of the target substance and the like using this substrate with the immobilized probe, blocking treatment may be conducted so that non-bound part of the substrate may not bind to the target substance and the like after the probe is immobilized to the solid phase surface. Blocking may be conducted, for example, by dipping the substrate in a 2% aqueous solution of bovine serum albumin for about two hours. However, an aqueous solution of bovine serum albumin is preferred for the effect of preventing the target substance from adsorbing to area other than the probe immobilization site of the substrate. This blocking step may be conducted according to need, and for example, when the sample is provided limitedly on each spot on the substrate with the immobilized probe and the sample does not substantially adhere to the part other than the spots, blocking does not need not to be conducted. The degree of adherence of the sample to the part other than the spots depends on the type of the substrate. Particularly, in the case that the substrate is glass or quartz, blocking treatment does not need to be conducted. However, when the probe immobilizing substance is not contained in the probe medium and the substrate has been subjected to probe immobilizing treatment, blocking treatment is preferably conducted.

The thus prepared probe immobilizing substrate may be made, for example, according to its use to have a plurality of spots containing the same probe or to have a plurality of spots containing different probes. Types, amounts and arrangements of the probe may vary according to need. Substrate with the immobilized probe on which probes are disposed in high density prepared by these methods are then used to detect the target substance or the sequencing determination of the target nucleotide sequence, and the like. For example, in an application for detecting a single-stranded nucleotide acid and the nucleotide sequence thereof is a known substance, which may be contained in the sample, a single-stranded nucleic acid having a nucleic sequence complementary to the nucleotide sequence of the target substance is used as a probe and a substrate with an immobilized probe having a plurality of spots including the above mentioned probe on the solid phase is prepared and then, providing a sample to each of the spots on the substrate with the immobilized probe and placing it under a condition so that the single-stranded nucleic acid of the target substrate may be hybridized with the probe, and then, whether hybridization proceeded or not is detected by way of a conventional method including fluorescence detection at each spot, thereby detecting the target substance in the sample.

In an application where the nucleotide sequence of the target substance (single-stranded nucleic acid) is specified, a plurality of candidates of the nucleotide sequence of the target substance are set and single-stranded nucleic acids complementary to each of the nucleotide sequence group are spotted as a probe on the substrate. Than the sample is provided on each of the spots and the substrate is placed to a condition so that the single-stranded nucleic acid of the target substrate may be hybridized with the probe, and then, whether hybridization proceeded or not is detected by way of a conventional method including fluorescence detection at each spot, thereby specifying the nucleotide sequence of the target substance (single-stranded nucleic acid). Examples of other applications of the substrate with the immobilized probe of the present invention include screening of specific nucleotide sequences which DNA binding protein can recognize, or screening of chemical substances having a property to bind to DNA.

Probe medium may comprise a probe and a substance which immobilizes the probe to the substrate as described above, but it is composed of a probe and a substance which immobilize the probe to the substrate which are respectively contained in a container and mixed when immobilized onto the substrate.

Examples of probe include single-stranded nucleic acid probe, single-stranded DNA probe, single-stranded RNA probe and single-stranded PNA probe. The amount of the probe present in the media is preferably adjusted to, for example, 2 mer to 500 mer, particularly 2 mer to 80 mer of the nucleic acid probe and to the level of 0.05 to 500 µmol/l, particularly 0.5 to 50 µmol/l, inview of the stability of nucleic acid probe as probe medium. These probes may have functional groups, and examples thereof include amino (NH₂) group, isocyanate (NCO) group, mercapto (SH) group and hydroxyl (OH) group. Preferred functional group is amino group taking into consideration the readiness of synthesizing probe functional group and mercapto group taking into consideration the reactivity of the probe functional group.

Container is preferably a sealable container so that other impurities will not be mixed into the probe stored in the container and should be openable so that it can be readily opened to allow the contents thereof to be mixed for immobilization. Opening of the container is not particularly limited. State of the probe when stored in the container is not particularly limited and may be liquid or powder. When the functional group is mercapto group and the like, it is preferably converted into powder by way of freeze drying method and the like taking into consideration the stability of the probe. The probe to be stored is preferably refrigerated for securing the stability of the probe but the storing condition may be varied depending on the term of storage, types of the probe and probe functional group. Accordingly, the probe may be stored in a container and then mixed when it is immobilized onto the substrate.

Examples of the substance having a functional group at the site which can bond to the probe and a functional group at the site which can bond to the substrate include primer, coupling agent, sealant, surface treatment agent, surface modification agent, etc. The functional groups which can bond to the substrate include epoxy (CHOCH₂) group, amino (NH₂) group, mercapto (SH) group, maleimide group, isocyanate (NCO) group, chloropropyl (C₃H₆Cl) group, etc. Particularly preferred functional groups which can bond to the substrate include epoxy group, maleimide group, isocyanate group, chloropropyl group taking into consideration the readiness of immobilization onto the substrate. The functional groups at the site which can bond to the substrate include, silanol (SiOH) group. Particularly silanol (SiOH) group is preferred taking into consideration the readiness of immobilization onto the substrate and reactivity. Silanol group may be formed by hydrolysis of an alkoxylsilyl group. The probe and the substance which immobilizes the probe onto the substrate can bond to each other in the media through a reaction, preferably through a chemical reaction between the functional groups to form a covalent bond.

Container is preferably a sealable container so that other impurities will not be mixed into and the substance will not be decrease by evaporation when the substance having a functional group at the site which can bond to the probe and a functional group at the site which can bond to the substrate is stored in the container and should be openable so that it can be readily opened to allow the contents thereof to be mixed for immobilization. Opening of the container is, however, not particularly limited. State of the probe when stored in the container is not particularly limited and may be liquid or powder. When a functional group at the site which can bond to the probe and a functional group at the site which can bond to the substrate are contained in different substances, each of such substances may be stored in a container, or may be stored in different containers, without any limitation but it preferable to store them suitable for forming probe medium. The state of the substance is not particularly limited and may be liquid or may be powder. It may be a solution after hydrolysis for forming silanol group which is one example of the functional groups used for immobilizing to the substrate. Storing may be effected at an ambient temperature to ensure the stability of the substance but can be varied depending on storing term or the like. Particularly, when a hydrolyzed solution is used, temperature is preferably maintained so that it does not increase. Further, pH during or after the hydrolysis is preferably adjusted depending on the functional group of the substance which immobilizes the probe to the substrate.

At the time when the probe is immobilized to the substrate, water may be added according to need while the probe and the substance having a functional group at the site which can bond to the probe and a functional group at the site which can bond to the substrate are mixed. Further, a substance which enables sufficient mixing to form probe medium, such as water or the like solvent, may be placed in another container and admixed when the probe medium is prepared.

Examples of water-soluble polymer material include polyvinyl alcohol (PVA), polyvinylpyrrolidone (PVP), paogen, carboxymethylcellulose(CMC), hydroxyethylcellulose(HEC), dextran and pullulane. Among these, polyvinyl alcohol (PVA) and polyvinylpyrrolidone (PVP) are preferable because they are commonly used material and stable to the probes. In case of commonly used polyvinyl alcohol (PVA), it is preferable to adjust the polymerization degree and saponification number depending according to need. Solubility of the polyvinyl alcohol in to the probe medium and viscosity of the probe medium can be adjusted by adjusting the polymerization degree and saponification number. Specifically, the polymerization degree and saponification number are adjusted to attain a suitable probe stability as long as they may not cause troubles in providing the probe medium on the substrate. However, when the polymerization degree or saponification number of polyvinyl alcohol is too high, solubility of polyvinyl alcohol into the probe medium decrease while viscosity thereof increases, and therefore, the probe medium becomes difficult to be readily dissolved when it is prepared as probe medium. To avoid these problems, the concentration of polyvinyl alcohol may be lowered but the decreased concentration also lowers probe stability, spot form properties and hygroscopic property, and therefore suitable selection of polyvinyl alcohol and adjustment of concentration are required. The concentration of polyvinyl alcohol in the probe medium is preferably adjusted to 0.001 to 1 wt% when stored in a container.

When it is difficult to be dissolved it may be previously dissolved and then mixed with the probe medium. Organic solvents such as ethanol and dimethylsulfoxide (DMSO) may be used for dissolving media, but water and ethanol are preferred taking into consideration of the solubility. Polyvinyl alcohol may be mixed with water and then warmed to rapidly dissolve undissolved contents. Preferably, it is mixed with the probe medium after filtering dissolved polyviniyl alcohol to remove the undissolved contents. The thus prepared polyvinyl alcohol is adjusted to the range of pH 6.0 to pH 9.0, particularly pH 7.0 to pH 8.0 taking into consideration the probe stability when mixed with the probe.

Container is preferably a sealable container so that other impurities will not be mixed into and the water-soluble polymer material will not be decrease by evaporation and should be openable so that it can be readily opened to allow the contents thereof to be mixed for immobilization. Storing may be effected at an ambient temperature to ensure the stability of the substance but can be varied depending on storing term or the like. Particularly, when a solution of a water-soluble polymer material is used, temperature is preferably maintained so that it does not increase. Further, pH during or after the hydrolysis is preferably adjusted depending on the functionally group of the substance which immobilizes the probe to the substrate.

Examples of high boiling solvent include ethylene glycol, propylene glycol, triethylene glycol, diethylene glycol, dipropylene glycol and thiodiglycol. Among these, thiodiglycol and dipropylene glycol are preferable because they are commonly used materials and suitable as spotting media. When a high boiling solvent is added it is preferred that the probe medium has suitable properties in spot form properties, hygroscopic property and drying property.

Container is preferably a sealable container so that other impurities will not be mixed into and the high boiling solvent will not be decrease by evaporation and should be openable so that it can be readily opened to allow the contents thereof to be mixed for immobilization. Storing may be effected at an ambient temperature to ensure the stability of the substance but can be varied depending on storing term or the like. Particularly, temperature is preferably maintained so that it does not increase. Further, pH during or after the hydrolysis is preferably adjusted depending on the functionally group of the substance which immobilizes the probe to the substrate.

At the time when the probe is immobilized to the substrate, water may be added according to need while the probe and the substance having a functional group at the site which can bond to the probe and a functional group at the site which can bond to the substrate are mixed. Further, a substance which enables sufficient mixing to form probe medium, such as water or the like solvent, may be placed in another container and admixed when the probe medium is prepared.

The present invention will be described further in detail below with reference to Examples.

### [Example 1]

### (1) Synthesis of probe

A single-stranded DNA probe was used as a probe capable of being specifically bound to a target material. Single-stranded DNA of SEQ ID NO: 1 was synthesized using a DNA automatic synthesizer, and an amino group was bound to the 5' terminal hydroxyl group of the single stranded DNA via a phosphate group and hexamethylene to prepare an octadecanomeric oligomer of Formula (1) for use in the following experiment.

5'H₂N-(CH₂)₆-O-PO₂-O-ACTGGCCGTCGTTTTACA3' (1)

(2) Material having functional group of site capable of being bound to probe and functional group of site capable of being bound on substrate (probe binding material)

For preparation of a material having a functional group of a site capable of being bound to a probe and a functional group of a site capable of being bound on a substrate, an aqueous solution of 500 µmol/l of silane coupling agent (trade name: KBM-403 manufactured by The Shin-Etsu Chemical Co., Ltd.) containing a silane compound (γ-glycidoxypropyltrimethoxy silane) having an epoxy group was stirred at a room temperature for 30 minutes to prepare a solution of probe binding material. The prepared solution of probe binding material was used in the following experiment.

### (3) Preparation of probe medium

The single-stranded DNA probe of Formula (1) was dissolved in a TE solution (10 mM Tris-HCl (pH8)/1 mM, aqueous solution of ethylenediaminetetraacetic acid (EDTA)) so that the final concentration was about 40 µmol/l, thereby preparing a solution of single-stranded DNA probe (accurate concentration was calculated from absorption intensity).

Then, polyvinyl alcohol (PVA) as a water-soluble polymer material was dissolved in pure water so that its concentration was 0.5 wt%). For dissolving the polyvinyl alcohol completely, the resulting solution was heated in a hot bath at 80°C while it was stirred for 60 minutes. After it was checked that no insoluble matter existed, the solution was subjected to filtering to prevent clogging of a nozzle in spotting, whereby a PVA solution was prepared.

100 µl of probe solution prepared as described above was prepared, and 100 µl of solution of probe binding material was dropped therein, and was mixed for 5 minutes. After the mixture was left to stand for 10 minutes, 700 µl of pure water and 100 µl of PVA solution prepared as described above were dropped therein, and were mixed for 5 minutes. After mixing, the mixture was left to stand for 30 minutes to prepare a probe medium.

### (4) Cleaning of substrate

A glass substrate (thickness: 1.1 mm) of 1 inch × 3 inch was placed in a cassette, and was submerged for 10 minutes in 1 mol/l of sodium hydroxide solution heated to 60°C in advance. Subsequently, they were sufficiently rinsed in flowing pure water to wash away sodium hydroxide stuck to the glass substrate and cassette. After rinsing sufficiently, the glass substrate together with the cassette was submerged in pure water, and was subjected to ultra sonic cleaning for 10 minutes. After the ultra sonic cleaning, they were sufficiently rinsed in flowing pure water to wash away particles stuck to the glass substrate and the cassette. The rinsed glass substrate was dried one by one with a nitrogen gas blow. For checking the level of cleanliness of the substrate, the contact angle of pure water on the substrate was measured. As a result, the contact angle was about 5° at every site of the substrate.

### (5) Spotting of probe medium

The probe medium prepared in the step (3) was loaded in an ink tank for a bubble jet printer (trade name: BJF850; manufactured by Canon Inc.) to be installed in a bubble jet head. The bubble jet printer (trade name: BJF850; manufactured by Canon Inc.) used here had been subjected to a modification so that printing on a plane plate could be done. Then, the glass substrate prepared in the step (4) was installed in this printer, and the probe medium was spotted on the glass substrate. Here, the distance between the liquid discharge surface of the bubble jet head and the liquid retention surface of the glass substrate was in the range of from 1.2 to 1.5 mm. After the spotting process was completed, the glass substrate was observed by a microscope, and as a result it was found that a matrix shaped spot array was formed on the surface of the glass substrate. In addition, the contact angles of pure water were measured on outer peripheries of spot formation sites. As a result, the angle of contact was about 5° in every site of the plate. From this result, it could be found that contaminations were not caused by the probe medium and the like except for spot formation sites. The glass plate after undergoing the spotting process was left to stand in a desiccator. A substrate with an immobilized probe was fabricated in this way.

### (6) Blocking treatment

The substrate with the immobilized probe fabricated in the step (5) was sufficiently washed with a 1M NaCl/50 mM phosphate buffer solution (pH 7.0) to wet the surface of the substrate with the immobilized probe, thereby making it possible to carry out a blocking treatment uniformly. Then, the glass substrate was submerged in an aqueous solution of 2% bovine serum albumin, and was then left to stand for 2 hours to carry out a blocking treatment.

### (7) Hybridization treatment

A single-stranded DNA having a base sequence complementary to the single-stranded DNA probe of Formula (1) described in the step (1) was synthesized in a DNA automatic synthesizer to obtain a single-stranded DNA probe labeled with rhodamine bound to the 5' terminal. This labeled singles-stranded DNA is dissolved in the 1M NaCl/50 mM phosphate buffer solution (pH 7.0) to obtain the 1 µM solution of the labeled DNA, and the substrate with the immobilized probe subjected to the blocking treatment, obtained in the step (6), was submerged in the solution, and was subjected to a hybridization treatment at a room temperature (25°C) for 3 hours. Thereafter; the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (8) Results

As a result of analyzing the results of fluorescence scanner evaluation in the step (7), the fluorescence intensity was 14830 at 532 nm on the spot of the DNA probe of Formula (1) completely matching with the labeled single-stranded DNA probe. In addition, the fluorescence intensity of an area other than the spot area of the DNA probe was observed and found to be 614. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### [Example 2]

### (1) Synthesis of probe

A single-stranded DNA probe was prepared just in the same way as Example 1 described above for use in the following experiment.

(2) Material having functional group of site capable of being bound to probe and functional group of site capable of being bound on substrate (probe binding material)

For preparation of a material having a functional group of a site capable of being bound to a probe and a functional group of a site capable of being bound on a substrate, a silane coupling agent (trade name: KBM-403 manufactured by The Shin-Etsu Chemical Co., Ltd.) containing a silane compound (y-glycidoxypropyltrimethoxy silane) having an epoxy group was prepared. This silane coupling agent was used in the following experiment.

### (3) Preparation of probe medium

A solution of sinqle-stranded DNA probe and a PVA solution were prepared just in the same way as Example 1 described above.

10 ml of probe solution prepared as described above was prepared, and 11 µl of the above silane coupling agent was dropped therein, and was thereafter mixed for 20 minutes. After the mixture was left to stand for 30 minutes, 5 ml of pure water was repeatedly dropped and mixed to drop total 80 ml of pure water. Then, 10 ml of PVA solution prepared as described above was dropped, and was mixed for 10 minutes. After mixing, the mixture was left to stand for 30 minutes to prepare a probe medium (accurate concentration was calculated from absorption intensity).

### (4) Cleaning of substrate

A substrate was cleaned just in the same way as Example 1 described above to prepare a glass substrate.

### (5) Spotting of probe medium

The probe medium prepared in the step (3) was spotted just in the same way as Example 1 described above to fabricate a substrate with an immobilized probe. After the spotting process was completed, the glass substrate was observed by a microscope, and as a result it was found that a matrix shaped spot array was formed on the surface of the glass substrate. The glass plate after undergoing the spotting process was left to stand in a desiccator. A substrate with an immobilized probe was fabricated in this way.

### (6) Blocking treatment

A blocking treatment was carried out just in the same way as Example 1 described above.

### (7) Hybridization treatment

A hybridization treatment was carried out just in the same way as Example 1 described above. Thereafter, the probe array was washed with a 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (8) Results

As a result of analyzing the results of fluorescence scanner evaluation in the step (7), the fluorescence intensity was 23400 at 532 nm on the spot of the DNA probe of Formula (1) completely matching with the labeled single-stranded DNA probe. In addition, the fluorescence intensity of an area other than the spot area of the DNA probe was observed and found to be 672. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### [Example 3]

### (1) Synthesis of probe

A single-stranded DNA probe was used as a probe capable of being specifically bound to a target material. Single-stranded nucleic acids of SEQ ID NO:1, SEQ ID NO:2, SEQ ID NO:3 and SEQ ID NO:4 were synthesized using a DNA automatic synthesizer. For the single-stranded DNA of SEQ ID NO:2, 3 and 4, single-stranded DNA with one base changed was defined as SEQ ID NO:2, single-stranded DNA with three bases changed was defined as SEQ ID NO:3, and single-stranded DNA with six bases changed was defined as SEQ ID NO:4 on the basis of SEQ ID NO:1 used in Example 1. An amino group was bound to the 5' terminal hydroxyl group of each of single stranded DNAs of SEQ ID NO: 1 to 4 via a phosphate group and hexamethylene to obtain an octadecanomeric oligomer of Formulae (1), (2), (3) and (4) for use in the following experiment.

5'H₂N-(CH₂)₆-O-PO₂-O-ACTGGCCGTTGTTTTACA3' (2)

5' H₂N-(CH₂)₆-O-PO₂-O-ACTGGCCGCTTTTTTACA3' (3)

5' H₂N-(CH₂)₆-O-PO₂-O-ACTGGCATCTTGTTTACA3' (4)

(2) Material having functional group of site capable of being bound to probe and functional group of site capable of being bound on substrate (probe binding material)

For preparation of a material having a functional group of a site capable of being bound to a probe and a functional group of a site capable of being bound on a substrate, an aqueous solution of 500 µmol/l of silane coupling agent (trade name: KBM-403 manufactured by The Shin-Etsu Chemical Co., Ltd.) containing a silane compound (γ-glycidoxypropyltrimethoxy silane) having an epoxy group was prepared. This silane coupling agent was used in the following experiment.

### (3) Preparation of probe medium

The single-stranded DNAs of SEQ ID NO:1 to 4 were used to prepare four types of probe medium in the same way as described in the step (3) of Example 2 described above (accurate concentration was calculated from absorption intensity).

### (4) Cleaning of substrate

A substrate was cleaned just in the same way as Example 1 described above to prepare a glass substrate.

### (5) Spotting of probe medium

The four probe mediums prepared in the step (3) were loaded in four ink tanks for a bubble jet printer (trade name: BJF850; manufactured by Canon Inc.), respectively, and each of the ink tanks was installed in a bubble jet head. The bubble jet printer (trade name: BJF850; manufactured by Canon Inc.) used here had been subjected to a modification so that printing on a plane plate could be done. Then, the glass substrate prepared in the step (4) was installed in this printer, and each of the four probe medium was spotted on each of four areas of 3 × 3 mm on the glass substrate. Furthermore, the pattern of spotting in each area was same as that of Example 1. Here, the distance between the liquid discharge surface of the bubble jet head and the liquid retention surface of the glass substrate was in the range of from 1.2 to 1.5 mm. After the spotting process was completed, the glass substrate was observed by a microscope, and as a result it was found that a matrix shaped spot array was formed on the surface of the glass substrate. The glass plate after undergoing the spotting process was left to stand in a desiccator. A substrate with an immobilized probe was fabricated in this way.

### (6) Blocking treatment

A Blocking treatment was carried out just in the same way as Example 1 described above.

### (7) Hybridization treatment

A hybridization treatment was carried out just in the same way as Example 1 described above. Thereafter, the probe array was washed with a 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (8) Results

As a result of analyzing the results of fluorescence scanner evaluation in the step (7), the fluorescence intensity was 14380 at 532 nm on the spot of the DNA probe of Formula (1) completely matching with the labeled single-stranded DNA probe. In contrast to this, for the spot of the DNA probe of Formula (2) having a one-base mismatch sequence, the fluorescence intensity was 8960. Also, for the spot of the DNA probe of Formula (3) having a three-base mismatch sequence, the fluorescence intensity was 2032, which was less than half of the fluorescence intensity of the completely matching DNA probe, and for the DNA probe of Formula (4) having a six-base mismatch, little fluorescence spot could be recognized where the fluorescence intensity at the site corresponding to the spot was 876. In addition, the fluorescence intensity of an area other than the spot area of the DNA probe was observed and found to be 637. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm. From the above, a fully complementary single stranded DNA could be detected specifically on the DNA array substrate.

### [Example 4]

### (1) Synthesis of probe

A single-stranded DNA probe was prepared just in the same way as Example 1 described above for use in the following experiment.

(2) Material having functional group of site capable of being bound to probe and functional group of site capable of being bound on substrate (probe binding material)

For preparation of a material having a functional group of a site capable of being bound to a probe and a functional group of a site capable of being bound on a substrate, a silane coupling agent (trade name: KBM-403 manufactured by The Shin-Etsu Chemical Co., Ltd.) containing a silane compound (γ-glycidoxypropyltrimethoxy silane) having an epoxy group was prepared. This silane coupling agent was used in the following experiment.

### (3) Preparation of probe medium

A solution of single-stranded DNA probe was prepared just in the same way as Example 1 described above.

10 ml of probe solution prepared as described above was prepared, and 11 µl of the above silane coupling agent was dropped therein, and was thereafter mixed for 20 minutes. After the mixture was left to stand for 30 minutes, 5 ml of solution containing 7.5 wt% of glycerin, 7.5 wt% of urine, and 7.5 wt% of thiodiglycol was repeatedly dropped and mixed while total 90 ml of pure water was dropped. Then they were mixed for 10 minutes. After mixing, the mixture was left to stand for 30 minutes to prepare a probe medium.

### (4) Cleaning of substrate

A substrate was cleaned just in the same way as Example 1 described above to prepare a glass substrate.

### (5) Spotting of probe medium

The probe medium prepared in the step (3) was spotted just in the same way as Example 1 described above to fabricate a substrate with an immobilized probe. After the spotting process was completed, the glass substrate was observed by a microscope, and as a result it was found that a matrix shaped spot array was formed on the surface of the glass substrate. The glass substrate after undergoing the spotting process was submerged in a container filled with a 1 M NaCl/50 mM phosphate buffer solution (pH 7.0), and was put in a refrigerator together with the container to be stored at a low temperature (4°C). The substrate with the immobilized probe was fabricated in this way.

### (6) Blocking treatment

The substrate with the immobilized probe fabricated in the step (5) was taken out from the 1 M NaCl/50 mM phosphate buffer solution (pH 7.0). Then, the glass substrate was submerged in a container filled with an aqueous solution of 2% bovine serum albumin, and was then left to stand for 2 hours to carry out a blocking treatment.

### (7) Hybridization treatment

A hybridization treatment was carried out just in the same way as Example 1 described above. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 40008 prepared by Axon Instruments, Inc.). For evaluating the fluorescence intensity, the laser power was set at 100%, and the PMT was set at 400 V.

### (8) Results

As a result of analyzing the results of fluorescence scanner evaluation in the step (7), the fluorescence intensity was 23400 at 532 nm on the spot of the DNA probe of Formula (1) completely matching with the labeled single-stranded DNA probe. In addition, the fluorescence intensity of an area other than the spot area of the DNA probe was observed and found to be 628. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### [Example 5]

### (1) Synthesis of probe

A single-stranded DNA probe was prepared just in the same way as Example 1 described above for use in the following experiment.

(2) Material having functional group of site capable of being bound to probe and functional group of site capable of being bound on substrate (probe binding material)

For preparation of a material having a functional group of a site capable of being bound to a probe and a functional group of a site capable of being bound on a substrate, an aqueous solution of 500 µmol/l of silane coupling agent (trade name: KBM-703 manufactured by The Shin-Etsu Chemical Co., Ltd.) containing a silane compound (γ-chloropropyltrimethoxy silane) having a chloropropyl group was pH-adjusted so that the pH of the solution was 4.0, and was thereafter stirred at a room temperature for 30 minutes to prepare a solution of probe immobilizing material. The prepared solution of probe immobilizing material was used in the following experiment.

### (3) Preparation of probe medium

A solution of single-stranded DNA probe was prepared just in the same way as Example 1 described above.

100 µl of probe solution prepared as described above was prepared, and 100 µl of solution of probe immobilizing material was dropped therein, and was mixed for 5 minutes. After the mixture was left to stand for 10 minutes, 800 µl of pure water was dropped therein, and was mixed for 5 minutes. After mixing, the mixture was left to stand for 30 minutes to prepare a probe medium.

### (4) Cleaning of substrate

A substrate was cleaned just in the same way as Example 1 described above to prepare a glass substrate.

### (5) Spotting of probe medium

The probe medium prepared in the step (3) was spotted just in the same way as Example 1 described above to fabricate a substrate with an immobilized probe. After the spotting process was completed, the glass substrate was observed by a microscope, and as a result it was found that a matrix shaped spot array was formed on the surface of the glass substrate. The glass substrate after undergoing the spotting process was submerged in a container filled with a 1 M NaCl/50 mM phosphate buffer solution (pH 7.0), and was put in a refrigerator together with the container to be stored at a low temperature (4°C). The substrate with the immobilized probe was fabricated in this way.

### (6) Blocking treatment

A blocking treatment was carried out just in the same way as Example 4 described above.

### (7) Hybridization treatment

A hybridization treatment was carried out just in the same way as Example 1 described above. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (8) Results

As a result of analyzing the results of fluorescence scanner evaluation in the step (7), the fluorescence intensity was 11320 at 532 nm on the spot of the DNA probe of Formula (1) completely matching with the labeled single-stranded DNA probe. In addition, the fluorescence intensity of an area other than the spot area of the DNA probe was observed and found to be 641. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### [Example 6]

### (1) Synthesis of probe

A single-stranded DNA probe was prepared just in the same way as Example 1 described above for use in the following experiment.

(2) Material having functional group of site capable of being bound to probe and functional group of site capable of being bound on substrate (probe binding material)

For preparation of a material having a functional group of a site capable of being bound to a probe and a functional group of a site capable of being bound on a substrate, a silane coupling agent (trade name: KBM-703 manufactured by The Shin-Etsu Chemical Co., Ltd.) containing a silane compound (γ-chloropropyltrimethoxy silane) having a chloropropyl group was prepared. This silane coupling agent was used in the following experiment.

### (3) Preparation of probe medium

A solution of single-stranded DNA probe and a PVA solution were prepared just in the same way as Example 1 described above.

10 ml of probe solution prepared as described above was prepared, and 10 µl of the above silane coupling agent was dropped therein, and was thereafter mixed for 20 minutes. After the mixture was left to stand for 30 minutes, 5 ml of pure water was repeatedly dropped and mixed to drop total 80 ml of pure water. Then, 10 ml of PVA solution prepared as described above was dropped, and was mixed for 10 minutes. After mixing, the mixture was left to stand for 30 minutes to prepare a probe medium.

### (4) Cleaning of substrate

A substrate was cleaned just in the same way as Example 1 described above to prepare a glass substrate.

### (5) Spotting of probe medium

The probe medium prepared in the step (3) was spotted just in the same way as Example 1 described above to fabricate a substrate with an immobilized probe. After the spotting process was completed, the glass substrate was observed by a microscope, and as a result it was found that a matrix shaped spot array was formed on the surface of the glass substrate. The glass plate after undergoing the spotting process was left to stand in a desiccator. A substrate with an immobilized probe was fabricated in this way.

### (6) Blocking treatment

A blocking treatment was carried out just in the same way as Example 1 described above.

### (7) Hybridization treatment

A hybridization treatment was carried out just in the same way as Example 1 described above. Thereafter, the probe array was washed with a 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (8) Results

As a result of analyzing the results of fluorescence scanner evaluation in the step (7), the fluorescence intensity was 15790 at 532 nm on the spot of the DNA probe of Formula (1) completely matching with the labeled single-stranded DNA probe. In addition, the fluorescence intensity of an area other than the spot area of the DNA probe was observed and found to be 608. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### [Example 7]

### (1) Synthesis of probe

A single-stranded DNA probe of Formula (1) was prepared just in the same way as Example 1 described above for use in the following experiment.

(2) Material having functional group of site capable of being bound to probe and functional group of site capable of being bound on substrate (probe binding material)

A material having a functional group of a site capable of being bound to a probe and a functional group of a site capable of being bound on a substrate was prepared just in the same way as Example 1 for use in the following experiment.

### (3) Preparation of probe medium

A probe medium was prepared just in the same way as Example 1 described above.

### (4) Cleaning of substrate

A substrate was cleaned just in the same way as Example 1 described above to prepare a glass substrate.

### (5) Spotting of probe medium

A substrate with an immobilized probe was fabricated just in the same way as Example 1 described above.

### (6) Blocking treatment

The substrate with the immobilized probe was sufficiently washed with a 1 M NaCl/50 mM phosphate buffer solution (pH 7.0) to wet the surface of the substrate with the immobilized probe, thereby making it possible to carry out a blocking treatment uniformly, without conducting the blocking treatment carried out in Example 1 described above.

### (7) Hybridization treatment

A hybridization treatment was carried out just in the same way as Example 1 described above. Thereafter, the probe array was washed with a 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (8) Results

As a result of analyzing the results of fluorescence scanner evaluation in the step (7), the fluorescence intensity was 23400 at 532 nm on the spot of the DNA probe of Formula (1) completely matching with the labeled single-stranded DNA probe. In addition, the fluorescence intensity of an area other than the spot area of the DNA probe was observed and found to be 658. When the spot of each DNA probe was observed with fluorescence, each spot appeared to be almost circular, and there was little difference in fluorescence intensity among spots obtained by spotting the same probe medium. There was little increase in fluorescence intensity associated with omission of the blocking treatment in the area other than the spot area. In addition, it was observed that the distances between adjacent spots were almost the same, and the spots were placed in a lattice form at intervals of about 200 µm.

### [Example 8]

### (1) Synthesis of probe

A single-stranded DNA probe was used as a probe capable of being specifically bound to a target material. A single-stranded nucleic acid of SEQ ID NO:5 was synthesized using a DNA automatic synthesizer, and a mercapto (SH) group was introduced into the terminal of the single-stranded nucleic acid by using Thiol-Modifier (manufactured by GlenResearch Co., Ltd.) during synthesis by the DNA automatic synthesizer. Subsequently, normal deprotection was performed, and the DNA was collected and purified by high performance liquid chromatography to obtain an oligomer of Formula (5) for use in the following experiment.

5'HS-(CH₂)₆-O-PO₂-O-ACTGGCCGTCGTTTTACA3' (5)

(2) Material having functional group of site capable of being bound to probe (probe binding material)

For preparing a material having a functional group of a site capable of being bound to a probe, an aqueous solution of 500 µmol/l of bivalent reagent (N-(6-maleimidocaproyloxy) sulfosuccinimide, sodium salt (trade name: sulfo-EMCS; manufactured by Dojindo LaboraLories Corporate, ltd.)) having a malcimide group and a hydroxysuccinimide ester group was stirred at a room temperature for 5 minutes to prepare a solution of probe binding material. The prepared solution of probe binding material was used in the following experiment.

(3) Material having functional group of site capable of being bound on substrate (substrate binding material)

For preparing a material having a functional group of a site capable of being bound on a substrate, an aqueous solution of 20 mmol/l of silane coupling agent (trade name: KBM-603 manufactured by The Shin-Etsu Chemical Co., Ltd.) containing a silane compound (N-β (aminoethyl) γ-aminopropyltrimethoxy silane) having an amino group was stirred at a room temperature for 30 minutes to prepare a solution of substrate binding material. The prepared solution of substrate binding material was used in the following experiment.

### (4) Preparation of probe medium

The single-stranded DNA probe of Formula (5) was dissolved in a TE solution (10 mM Tris-HCl (pH8)/1 mM, aqueous solution of ethylenediaminetetraacetic acid (EDTA)) so that the final concentration was about 40 µmol/l, thereby preparing a solution of single-stranded DNA probe (accurate concentration was calculated from absorption intensity).

100 µl of probe solution prepared as described above was prepared, and 100 µl of solution of probe binding material prepared in the step (2) was dropped therein, and was thereafter mixed for 5 minutes. Then, 100 µl of solution of substrate binding material prepared in the step (3) was dropped therein, and was thereafter mixed for 5 minutes. After the mixture was left to stand for 10 minutes, 700 µl of solution containing 7.5 wt% of glycerin, 7.5 wt% of urine, and 7.5 wt% of thiodiglycol was dropped, and was mixed for 5 minutes. After mixing, the mixture was left to stand for 30 minutes to prepare a probe medium.

### (5) Cleaning of substrate

A glass substrate (thickness: 1.1 mm) of 1 inch × 3 inch was placed in a cassette, and was submerged for 10 minutes in 1 mol/l of sodium hydroxide solution heated to 60°C in advance. Subsequently, they were sufficiently rinsed in flowing pure water to wash away sodium hydroxide stuck to the glass substrate and cassette. After rinsing sufficiently, the glass substrate together with the cassette was submerged in pure water, and was subjected to ultra sonic cleaning for 10 minutes. After the ultra sonic cleaning, they were sufficiently rinsed in flowing pure water to wash away particles stuck to the glass substrate and the cassette. The rinsed glass substrate was dried one by one with a nitrogen gas blow. For checking the level of cleanliness of the substrate, the contact angle of pure water on the substrate was measured. As a result, the contact angle was about 5° at every site of the substrate.

### (6) Spotting of probe medium

The probe medium prepared in the step (4) was loaded in an ink tank for a bubble jet printer (trade name: BJF850; manufactured by Canon Inc.) to be installed in a bubble jet head. The bubble jet printer (trade name: BJF850; manufactured by Canon Inc.) used here had been subjected to a modification so that printing on a plane plate could be done. Then, the glass substrate prepared in the step (5) was installed in this printer, and the probe medium was spotted on the glass substrate. Here, the distance between the liquid discharge surface of the bubble jet head and the liquid retention surface of the glass substrate was in the range of from 1.2 to 1.5 mm. After the spotting process was completed, the glass substrate was observed by a microscope, and as a result it was found that a matrix shaped spot array was formed on the surface of the glass substrate. In addition, the contact angles of pure water were measured on outer peripheries of spot formation sites. As a result, the angle of contact was about 5° in every site of the plate. From this result, it could be found that contaminations were not caused by the probe medium and the like except for spot formation sites. The glass substrate after undergoing the spotting process was submerged in a container filled with a 1 M NaCl/50 mM phosphate buffer solution (pH 7.0), and was put in a refrigerator together with the container to be stored at a low temperature (4°C). The substrate with the immobilized probe was fabricated in this way.

### (7) Blocking treatment

The substrate with the immobilized probe fabricated in the step (6) was taken out from the 1M NaCl/50 mM phosphate buffer solution (pH 7.0). Then, the glass substrate was submerged in a container filled with an aqueous solution of 2% bovine serum albumin, and was then left to stand for 2 hours to carry out a blocking treatment.

### (8) Hybridization treatment

A single-stranded DNA having a base sequence complementary to the single-stranded DNA probe of Formula (5) described in the step (1) was synthesized in a DNA automatic synthesizer to obtain a single-stranded DNA probe labeled with rhodamine bound to the 5' terminal. This labeled singles-stranded DNA is dissolved in the 1M NaCl/50 mM phosphate buffer solution (pH 7.0) to obtain the 1µM solution of the labeled DNA, and the substrate with the immobilized probe subjected to the blocking treatment, obtained in the step (7), was submerged in the solution, and was subjected to a hybridization treatment at a room temperature (25°C) for 2 hours. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (9) Results

As a result of analyzing the results of fluorescence scanner evaluation in the step (8), the fluorescence intensity was 25730 at 532 nm on the spot of the DNA probe of Formula (5) completely matching with the labeled single-stranded DNA probe. In addition, the fluorescence intensity of an area other than the spot area of the DNA probe was observed and found to be 639. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### [Example 9]

### (1) Synthesis of probe

A single-stranded DNA probe of Formula (5) was prepared just in the same way as Example 8 described above for use in the following experiment.

(2) Material having functional group of site capable of being bound to probe (probe binding material)

For preparing a material having a functional group of a site capable of being bound to a probe, a bivalent reagent (N-(6-Maleimidocaproyloxy) sulfosuccinimide, sodium salt (trade name: sulfo-EMCS; manufactured by Dojindo Laboratries Corporate, ltd.)) having a maleimide group was prepared. This probe binding material was used in the following experiment.

(3) Material having functional group of site capable of being bound on substrate (substrate binding material)

A solution of substrate binding material was prepared just in the same way as Example 8. The prepared solution of substrate binding material was used in the following experiment.

### (4) Preparation of probe medium

A solution of single-stranded DNA probe was prepared just in the same way as Example 8 described above.

10 ml of probe solution prepared as described above was prepared, and about 2.1 mg of probe binding material described above was weighed and added in the solution, and was thereafter mixed for 5 minutes. After the mixture was left to stand for 30 minutes, 10 ml of pure water was repeatedly dropped in the mixed medium of probe and probe binding material and mixed to drop therein total 80 ml of pure water. Then, 10 ml of solution of substrate binding material prepared in the step (3) was dropped and mixed for 5 minutes. After mixing, the mixture was left to stand for 30 minutes to prepare a probe medium.

### (5) Cleaning of substrate

A substrate was cleaned just in the same way as Example 8 described above to prepare a glass substrate.

### (6) Spotting of probe medium

The probe medium prepared in the step (4) was spotted just in the same way as Example 8 described above to fabricate a substrate with an immobilized probe. After the spotting process was completed, the glass substrate was observed by a microscope, and as a result it was found that a matrix shaped spot array was formed on the surface of the glass substrate. The glass substrate after undergoing the spotting process was submerged in a container filled with a 1 M NaCl/50 mM phosphate buffer solution (pH 7.0), and was put in a refrigerator together with the container to be stored at a low temperature (4°C). The substrate with the immobilized probe was fabricated in this way.

### (7) Blocking treatment

A blocking treatment was carried out just in the same way as Example 8 described above.

### (8) Hybridization treatment

A hybridization treatment was carried out just in the same way as Example 8 described above. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (9) Results

As a result of analyzing the results of fluorescence scanner evaluation in the step (8), the fluorescence intensity was 25100 at 532 nm on the spot of the DNA probe of formula (5) completely matching with the labeled single-stranded DNA probe. In addition, the fluorescence intensity of an area other than the spot area of the DNA probe was observed and found to be 769. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### [Example 10]

### (1) Synthesis of probe

A single-stranded DNA probe was used as a probe capable of being specifically bound to a target material. Single-stranded nucleic acids of SEQ ID NO:5, SEQ ID NO:6, SEQ ID NO:7 and SEQ ID NO:8 were synthesized using a DNA automatic synthesizer. For the single-stranded DNA of SEQ ID NO:6, 7 and 8, single-stranded DNA with one base changed was defined as SEQ ID NO:6, single-stranded DNA with three bases changed was defined as SEQ ID NO:7, and single-stranded DNA with six bases changed was defined as SEQ ID NO:8 on the basis of SEQ ID NO:5 used in Example 8. In addition, a thiol (SH) group was introduced into the terminal of each of single-stranded DNAs of SEQ ID NO:5 to 8 by using Thiol-Modifier (manufactured by GlenResearch Ltd.) during synthesis by the DNA automatic synthesizer. Subsequently, normal deprotection was performed, and the DNAs were collected and purified by high performance liquid chromatography to obtain oligomers of Formula (6) (7) and (8) for use in the following experiment.

5' HS- (CH₂)₆-O-PO₂-O-ACTGGCCGTTGTTTTACA3' (6)

5'HS-(CH₂)₆-O-PO₂-O-ACTGGCCGCTTTTTTACA3' (7)

5'HS- (CH₂)₆-O-PO₂-O-ACTGGCATCTTGTTTACA3' (8)

(2) Material having functional group of site capable of being bound to probe (probe binding material)

A solution of probe binding material was prepared in just the same way as Example 8 described above. The prepared solution of probe binding material was used in the following experiment.

(3) Material having functional group of site capable of being bound on substrate (substrate binding material)

A solution of substrate binding material was prepared just in the same way as Example 8. The prepared solution of substrate binding material was used in the following experiment.

### (4) Preparation of probe medium

A solution of single-stranded DNA probe was prepared just in the same way as Example 8 described above.

The single-stranded DNAs of SEQ ID NO:5 to 8 were used to prepare four types of probe medium in the same way as described in the step (4) of Example 8 described above (accurate concentration was calculated from absorption intensity).

### (5) Cleaning of substrate

A substrate was cleaned just in the same way as Example 8 described above to prepare a glass substrate.

### (6) Spotting of probe medium

The four probe mediums prepared in the step (4) were loaded in four ink tanks for a bubble jet printer (trade name: BJF850; manufactured by Canon Inc.), respectively, and each of the ink tanks was installed in a bubble jet head. The bubble jet printer (trade name: BJF850; manufactured by Canon Inc.) used here had been subjected to a modification so that printing on a plane plate could be done. Then, the glass substrate prepared in the step (5) was installed in this printer, and each of the four probe medium was spotted on each of four areas of 3 × 3 mm on the glass substrate. Furthermore, the pattern of spotting in each area was same as that of Example 8. Here, the distance between the liquid discharge surface of the bubble jet head and the liquid retention surface of the glass substrate was in the range of from 1.2 to 1.5 mm. After the spotting process was completed, the glass substrate was observed by a microscope, and as a result it was found that a matrix shaped spot array was formed on the surface of the glass substrate. The glass substrate after undergoing the spotting process was submerged in a container filled with a 1 M NaCl/50 mM phosphate buffer solution (pH 7.0), and was put in a refrigerator together with the container to be stored at a low temperature (4°C). A substrate with an immobilized probe with four types of probes immobilized thereon was fabricated in this way.

### (7) Blocking treatment

A blocking treatment was carried out just in the same way as Example 8 described above.

### (8) Hybridization treatment

A single-stranded DNA having a base sequence complementary to the single-stranded DNA probe of Formula (5) described in Example 8 described above was synthesized in a DNA automatic synthesizer to obtain a single-stranded DNA probe labeled with rhodamine bound to the 5' terminal. This labeled singles-stranded DNA is dissolved in the 1M NaCl/50 mM phosphate buffer solution (pH 7.0) to obtain 1µM solution of the labeled DNA, and the substrate with the immobilized probe subjected to the blocking treatment, obtained in the step (7), was submerged in the solution, and was subjected to a hybridization treatment at a room temperature (25°C) for 2 hours. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (9) Results

As a result of analyzing the results of fluorescence scanner evaluation in the step (8), the fluorescence intensity was 26120 at 532 nm on the spot of the DNA probe of Formula (5) completely matching with the labeled single-stranded DNA probe. In contrast to this, for the spot of the DNA probe of Formula (6) having a one-base mismatch sequence, the fluorescence intensity was 12870. Also, for the spot of the DNA probe of Formula (7) having a three-base mismatch sequence, the fluorescence intensity was 4220, which was less than half of the fluorescence intensity of the completely matching DNA probe, and for the DNA probe of Formula (8) having a six-base mismatch, little fluorescence spot could be recognized where the fluorescence intensity at the site corresponding to the spot was 791. In addition, the fluorescence intensity of an area other than the spot area of the DNA probe was observed and found to be 675. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm. From the above, a fully complementary single stranded DNA could be detected specifically on the DNA array substrate.

### [Example 11]

### (1) Synthesis of probe

A single-stranded DNA probe of Formula (5) was prepared just in the same way as Example 8 described above for use in the following experiment.

(2) Material having functional group of site capable of being bound to probe (probe binding material)

A solution of probe binding material was prepared in just the same way as Example 8 described above. The prepared solution of probe binding material was used in the following experiment.

(3) Material having functional group of site capable of being bound on substrate (substrate binding material)

A solution of substrate binding material was prepared just in the same way as Example 8. The prepared solution of substrate binding material was used in the following experiment.

### (4) Preparation of probe medium

A probe medium was prepared just in the same way as Example 8 described above.

### (5) Cleaning of substrate

A substrate was cleaned just in the same way as Example 8 described above to prepare a glass substrate.

### (6) Spotting of probe medium

A substrate with an immobilized probe was fabricated just in the same way as Example 8 described above.

### (7) Blocking treatment

The substrate was washed with a 1 M NaCl/50 mM phosphate buffer solution (pH 7.0), thereby making it possible to carry out a blocking treatment uniformly, without conducting the blocking treatment carried out in Example 8 described above.

### (8) Hybridization treatment

A hybridization treatment was carried out just in the same way as Example 8 described above. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (9) Results

As a result of analyzing the results of fluorescence scanner evaluation in the step (8), the fluorescence intensity was 24380 at 532 nm on the spot of the DNA probe of Formula (5) completely matching with the labeled single-stranded DNA probe. In addition, the fluorescence intensity of an area other than the spot area of the DNA probe was observed and found to be 783. When the spot of each DNA probe was observed with fluorescence, each spot appeared to be almost circular, and there was little difference in fluorescence intensity among spots obtained by spotting the same probe medium. There was little increase in fluorescence intensity associated with omission of the blocking treatment in the area other than the spot area. In addition, it was observed that the distances between adjacent spots were almost the same, and the spots were placed in a lattice form at intervals of about 200 µm.

### [Example 12]

### (1) Synthesis of probe

A single-stranded DNA probe was used as a probe capable of being specifically bound to a target material. Single-stranded DNA of SEQ ID NO:9 was synthesized using a DNA automatic synthesizer, and an amino group was bound to the 5' terminal hydroxyl group of the single stranded DNA via a phosphate group and hexamethylene to prepare an octadecanomeric oligomer of Formula (9) for use in the following experiment.

5' H₂N-(CH₂)₆-O-PO₂-O-ACTGGCCGTCGTTTTACA3' (9)

(2) Material having functional group of site capable of being bound to probe (probe binding material)

For preparing a material having a functional group of a site capable of being bound to a probe, an aqueous solution of 500 µmol/l of bivalent reagent (N-(8-Maleimidocaproyloxy) sulfosuccinimide, sodium salt (trade name: sulfo-HMCS; manufactured by Dojindo Laboratries Corporate, ltd.)) having a maleimide group was stirred at a room temperature for 5 minutes to prepare a solution of probe binding material. The prepared solution of probe binding material was used in the following experiment.

(3) Material having functional group of site capable of being bound on substrate (substrate binding material)

For preparing a material having a functional group of a site capable of being bound on a substrate, an aqueous solution of 20 mmol/l of silane coupling agent (trade name: KBM-802 manufactured by The Shin-Etsu Chemical Co., Ltd.) containing a silane compound (γ-mercaptopropylmethyldimethoxy silane) having a mercapto group was stirred at a room temperature for 30 minutes to prepare a solution of substrate binding material. The prepared solution of substrate binding material was used in the following experiment.

### (4) Preparation of probe medium

The single-stranded DNA probe of Formula (9) was dissolved in a TE solution (10 mM Tris-HCI (pH8)/1 mM, aqueous solution of ethylenediaminetetraacetic acid (EDTA)) so that the final concentration was about 40 µmol/l, thereby preparing a solution of single-stranded DNA probe (accurate concentration was calculated from absorption intensity).

Then, polyvinyl alcohol (PVA) as a water-soluble polymer material was dissolved in pure water so that its concentration was 0.5 wt%). For dissolving the polyvinyl alcohol completely, the resulting solution was heated in a hot bath at 80°C while it was stirred for 60 minutes. After it was checked that no insoluble matter existed, the solution was subjected to filtering to prevent clogging of a nozzle in spotting, whereby a PVA solution was prepared.

100 µl of probe solution prepared as described above was prepared, and 100 µl of solution of probe binding material prepared in the step (2) was dropped therein, and was thereafter mixed for 5 minutes. Then, 100 µl of solution of substrate binding material prepared in the step (3) was dropped therein, and was thereafter mixed for 5 minutes. After the mixture was loft to stand for 10 minutes, 600 µl of pure water and 100 µl of PVA solution prepared as described above were dropped therein, and were mixed for 5 minutes. After mixing, the mixture was left to stand for 30 minutes to prepare a probe medium.

### (5) Cleaning of substrate

A substrate was cleaned just in the same way as Example 8 described above to prepare a glass substrate.

### (6) Spotting of probe medium

The probe medium prepared in the above step (4) was loaded in an ink tank for a bubble jet printer (trade name: BJF850; manufactured by Canon Inc.) to be installed in a bubble jet head. The bubble jet printer (trade name: BJF850; manufactured by Canon Inc.) used here had been subjected to a modification so that printing on a plane plate could be done. Then, the glass substrate prepared in the above step (5) was installed in this printer, and the probe medium was spotted on the glass substrate. Here, the distance between the liquid discharge surface of the bubble jet head and the liquid retention surface of the glass substrate was in the range of from 1.2 to 1.5 mm. After the spotting process was completed, the glass substrate was observed by a microscope, and as a result it was found that a matrix shaped spot array was formed on the surface of the glass substrate. In addition, tho contact angles of pure water were measured on outer peripheries of spot formation sites. As a result, the angle of contact was about 5° in every site of the plate. From this result, it could be found that contaminations were not caused by the probe medium and the like except for spot formation sites. The glass plate after undergoing the spotting process was left to stand in a desiccator. A substrate with an immobilized probe was fabricated in this way.

### (7) Blocking treatment

The substrate with the immobilized probe fabricated in the step (5) was sufficiently washed with a 1M NaCl/50 mM phosphate buffer solution (pH 7.0) to wet the surface of the substrate with the immobilized probe, thereby making it possible to carry out a blocking treatment uniformly. Then, the glass substrate was submerged in an aqueous solution of 2% bovine serum albumin, and was then left to stand for 2 hours to carry out a blocking treatment.

### (8) Hybridization treatment

A single-stranded DNA having a base sequence complementary to the single-stranded DNA probe of Formula (9) described above was synthesized in a DNA automatic synthesizer to obtain a single-stranded DNA probe labeled with rhodamine bound to the 5' terminal. This labeled singles-stranded DNA is dissolved in the 1M NaCl/50 mM phosphate buffer solution (pH 7.0) to obtain the 1 µM solution of the labeled DNA, and the substrate with the immobilized probe subjected to the blocking treatment, obtained in the step (7), was submerged in the solution, and was subjected to a hybridization treatment at a room temperature (25°C) for 2 hours. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (9) Results

As a result of analyzing the results of fluorescence scanner evaluation in the step (8), the fluorescence intensity was 18710 at 532 nm on the spot of the DNA probe of Formula (5) completely matching with the labeled single-stranded DNA probe. In addition, the fluorescence intensity of an area other than the spot area of the DNA probe was observed and found to be 664. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### [Example 13]

### (1) Synthesis of probe

A single-stranded DNA probe of Formula (5) was prepared just in the same way as Example 12 described above for use in the following experiment.

(2) Material having functional group of site capable of being bound to probe (probe binding material)

A solution of probe binding material was prepared in just the same way as Example 12 described above. The prepared solution of probe binding material was used in the following experiment.

(3) Material having functional group of site capable of being bound on substrate (substrate binding material)

A solution of substrate binding material was prepared just in the same way as Example 12. The prepared solution of substrate binding material was used in the following experiment.

### (4) Preparation of probe medium

A solution of single-stranded DNA probe and a PVA solution were prepared just in the same way as Example 12 described above.

10 ml of probe solution prepared as described above was prepared, and about 2.1 mg of probe binding material described above was weighed and added in the solution, and was thereafter mixed for 5 minutes. After the mixture was left to stand for 30 minutes, 10 ml of pure water was repeatedly dropped in the mixed medium of probe and probe binding material and mixed to drop therein total 70 ml of pure water. Then, 10 ml of solution of substrate binding material prepared in the step (3) was dropped and mixed for 5 minutes. After mixing, the mixture was left to stand for 30 minutes, and thereafter 10 ml of PVA solution prepared as described above was dropped therein and mixed for 5 minutes. After the mixing was completed, the mixture was left to stand for 30 minutes to prepare a probe medium.

### (5) Cleaning of substrate

A substrate was cleaned just in the same way as Example 8 described above to prepare a glass substrate.

### (6) Spotting of probe medium

The probe medium prepared in the step (4) was spotted just in the same way as Example 8 described above to fabricate a substrate with an immobilized probe. After the spotting process was completed, the glass substrate was observed by a microscope, and as a result it was found that a matrix shaped spot array was formed on the surface of the glass substrate. The glass plate after undergoing the spotting process was left to stand in a desiccator. A substrate with an immobilized probe was fabricated in this way.

### (7) Blocking treatment

A blocking treatment was carried out just in the same way as Example 12 described above.

### (8) Hybridization treatment

A hybridization treatment was carried out just in the same way as Example 12 described above. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (9) Results

As a result of analyzing the results of fluorescence scanner evaluation in the step (8), the fluorescence intensity was 17970 at 532 nm on the spot of the DNA probe of Formula (5) completely matching with the labeled single-stranded DNA probe. In addition, the fluorescence intensity of an area other than the spot area of the DNA probe was observed and found to be 723. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### [Example 14]

### (1) Synthesis of probe

A single-stranded DNA probe of Formula (9) was prepared just in the same way as Example 12 described above for use in the following experiment.

(2) Material having functional group of site capable of being bound to probe (probe binding material)

For preparing a material having a functional group of a site capable of being bound to a probe, an aqueous solution of 500 µmol/l of bivalent reagent (N-(6-Maleimidocaproyloxy) sulfosuccinimide, sodium salt (trade name: sulfo-EMCS; manufactured by Dojindo Laboratories, ltd.)) having a maleimide group and a hydroxysuccinimide group was stirred at a room temperature for 5 minutes to prepare a solution of probe binding material. The prepared solution of probe binding material was used in the following experiment.

(3) Material having functional group of site capable of being bound on substrate (substrate binding material)

For preparing a material having a functional group of a site capable of being bound on a substrate, an aqueous solution of 20 mmol/l of silane coupling agent (trade name: KBM-603 manufactured by The Shin-Etsu Chemical Co., Ltd.) containing a silane compound (N-β (aminoethyl) γ-aminopropyltrimethoxy silane) having an amino group was stirred at a room temperature for 30 minutes to prepare a solution of substrate binding material. The prepared solution of substrate binding material was used in the following experiment.

### (4) Preparation of probe medium

The single-stranded DNA probe of Formula (9) was dissolved in a TE solution (10 mM Tris-HCI (pH8)/1 mM, aqueous solution of ethylenediaminetetraacetic acid (EDTA)) so that the final concentration was about 40 µmol/l, thereby preparing a solution of single-stranded DNA probe (accurate concentration was calculated from absorption intensity).

100 µl of probe solution prepared as described above was prepared, and 100 µl of solution of probe binding material prepared in the step (2) was dropped therein, and was thereafter mixed for 5 minutes. Then, 100 µl of solution of substrate binding material prepared in the step (3) was dropped therein, and was thereafter mixed for 5 minutes. After the mixture was left to stand for 10 minutes, 700 µl of solution containing 7.5 wt% of glycerin, 7.5 wt% of urine, and 7.5 wt% of thiodiglycol was dropped, and was mixed for 5 minutes. After mixing, the mixture was left to stand for 30 minutes to prepare a probe medium.

### (5) Cleaning of substrate

A glass substrate (thickness: 1.1 mm) of 1 inch × 3 inch was placed in a cassette, and was submerged for 10 minutes in 1 mol/l of sodium hydroxide solution heated to 60°C in advance. Subsequently, they were sufficiently rinsed in flowing pure water to wash away sodium hydroxide stuck to the glass substrate and cassette. After rinsing sufficiently, the glass substrate together with the cassette was submerged in pure water, and was subjected to ultra sonic cleaning for 10 minutes. After the ultra sonic cleaning, they were sufficiently rinsed in flowing pure water to wash away particles stuck to the glass substrate and the cassette. The rinsed glass substrate was dried one by one with a gas blow. For checking the level of cleanliness of the substrate, the contact angle of pure water on the substrate was measured. As a result, the contact angle was about 5° at every site of the substrate.

### (6) Spotting of probe medium

The probe medium prepared in the step (4) was loaded in an ink tank for a bubble jet printer (trade name: BJF850; manufactured by Canon Inc.) to be installed in a bubble jet head. The bubble jet printer (trade name: BJF850; manufactured by Canon Inc.) used here had been subjected to a modification so that printing on a plane plate could be done. Then, the glass substrate prepared in the step (5) was installed in this printer, and the probe medium was spotted on the glass substrate. Here, the distance between the liquid discharge surface of the bubble jet head and the liquid retention surface of the glass substrate was in the range of from 1.2 to 1.5 mm. After the spotting process was completed, the glass substrate was observed by a microscope, and as a result it was found that a matrix shaped spot array was formed on the surface of the glass substrate. In addition, the contact angles of pure water were measured on outer peripheries of spot formation sites. As a result, the angle of contact was about 5° in every site of the plate. From this result, it could be found that contaminations were not caused by the probe medium and the like except for spot formation sites. The glass substrate after undergoing the spotting process was submerged in a container filled with a 1 M NaCl/50 mM phosphate buffer solution (pH 7.0), and was put in a refrigerator together with the container to be stored at a low temperature (4°C) . The substrate with the immobilized probe was fabricated in this way.

### (7) Blocking treatment

The substrate with the immobilized probe fabricated in the step (6) was taken out from the 1M NaCl/50 mM phosphate buffer solution (pH 7.0). Then, the glass substrate was submerged in a container filled with an aqueous solution of 2% bovine serum albumin, and was then left to stand for 2 hours to carry out a blocking treatment.

### (8) Hybridization treatment

A single-stranded DNA having a base sequence complementary to the single-stranded DNA probe of Formula (9) described in the step (1) was synthesized in a DNA automatic synthesizer to obtain a single-stranded DNA probe labeled with rhodamine bound to the 5' terminal. This labeled singles-stranded DNA is dissolved in the 1M NaCl/50 mM phosphate buffer solution (pH 7.0) to obtain the 1µM solution of the labeled DNA, and the substrate with the immobilized probe subjected to the blocking treatment, obtained in the step (7), was submerged in the solution, and was subjected to a hybridization treatment at a room temperature (25°C) for 2 hours. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (9) Results

As a result of analyzing the results of fluorescence scanner evaluation in the step (8), the fluorescence intensity was 25730 at 532 nm on the spot of the DNA probe of Formula (9) completely matching with the labeled single-stranded DNA probe. In addition, the fluorescence intensity of an area other than the spot area of the DNA probe was observed and found to be 639. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### [Example 15]

### (1) Synthesis of probe

A single-stranded DNA probe was prepared just in the same way as Example 1 described above for use in the following experiment. The single-stranded DNA probe was distributed among micro tubes before being used. After the single-stranded DNA probe was distributed among micro tubes so that each micro tube contained 17.53 nmole of single-stranded DNA probe, they were subjected to dry-up drying to prepare the single-stranded DNA probe.

(2) Material having functional group of site capable of being bound to probe and functional group of site capable of being bound on substrate (probe binding material)

For preparation of a material having a functional group of a site capable of being bound to a probe and a functional group of a site capable of being bound on a substrate, a silane coupling agent (trade name: KBM-403 manufactured by The Shin-Etsu Chemical Co., Ltd.) containing a silane compound (γ-glycidoxypropyltrimethoxy silane) having an epoxy group was used. The epoxy silane coupling agent was used as a solution of probe binding material in the following experiment.

### (3) Preparation of probe medium

10 µl of epoxy silane coupling agent as a probe binding material was dropped and mixed in the micro tube containing the distributed single-stranded DNA probe of the Formula (1) and subjected to dry-up drying. Then, 50 µl of pure water was dropped and mixed therein. After mixing sufficiently for 5 minutes, the mixture was left to stand for 30 minutes.

Then, 500 µl of ethylene glycol and 500 µl of ethylene alcohol as high boiling solvents were dropped in the above micro tube, and were thereafter mixed for 1 minute.

Then, 100 µl of aqueous solution of 0.5 wt% of polyvinyl alcohol (PVA) as a water-soluble polymer material was dropped therein and thereafter mixed for 1 minute. Finally, pure water was dropped therein so that the total amount of probe material was 2000 µl, and was mixed. Thereafter, the mixture was left to stand for 60 minutes to prepare the probe medium.

### (4) Cleaning of substrate

A glass substrate (thickness: 1.1 mm) of 1 inch × 3 inch was placed in a cassette, and was submerged for 10 minutes in 1 mol/l of sodium hydroxide solution heated to 60°C in advance. Subsequently, they were sufficiently rinsed in flowing pure water to wash away sodium hydroxide stuck to the glass substrate and cassette. After rinsing sufficiently, the glass substrate together with the cassette was submerged in pure water, and was subjected to ultra sonic cleaning for 10 minutes. After the ultra sonic cleaning, they were sufficiently rinsed in flowing pure water to wash away particles stuck to the glass substrate and the cassette. The rinsed glass substrate was dried one by one with a nitrogen gas blow. For checking the level of cleanliness of the substrate, the contact angle of pure water on the substrate was measured. As a result, the contact angle was about 5° at every site of the substrate.

### (5) Spotting of probe medium

The probe medium prepared in the step (3) was loaded in an ink tank for a bubble jet printer (trade name: BJF850; manufactured by Canon Inc.) to be installed in a bubble jet head. The bubble jet printer (trade name: BJF850; manufactured by Canon Inc.) used here had been subjected to a modification so that printing on a plane plate could be done. Then, the glass substrate prepared in the step (4) was installed in this printer, and the probe medium was spotted on the glass substrate. Here, the distance between the liquid discharge surface of the bubble jet head and the liquid retention surface of the glass substrate was in the range of from 1.2 to 1.5 mm. After the spotting process was completed, the glass substrate was observed by a microscope, and as a result it was found that a matrix shaped spot array was formed on the surface of the glass substrate. In addition, the contact angles of pure water were measured on outer peripheries of spot formation sites. As a result, the angle of contact was about 5° in every site of the plate. From this result, it could be found that contaminations were not caused by the probe medium and the like except for spot formation sites. The glass plate after undergoing the spotting process was left to stand in a desiccator. A substrate with an immobilized probe was fabricated in this way.

### (6) Blocking treatment

The substrate with the immobilized probe fabricated in the step (5) was sufficiently washed with a 1M NaCl/50 mM phosphate buffer solution (pH 7.0) to wet the surface of the.substrate with the immobilized probe, thereby making it possible to carry out a blocking treatment uniformly. Then, the glass substrate was submerged in an aqueous solution of 2% bovine serum albumin, and was then left to stand for 2 hours to carry out a blocking treatment.

### (7) Hybridization treatment

A single-stranded DNA having a base sequence complementary to the single-stranded DNA probe of Formula (1) described in the step (1) was synthesized in a DNA automatic synthesizer to obtain a single-stranded DNA probe labeled with rhodamine bound to the 5' terminal. This labeled singles-stranded DNA is dissolved in the 1M NaCl/50 mM phosphate buffer solution (pH 7.0) to obtain the 1 µM solution of the labeled DNA, and the substrate with the immobilized probe subjected to the blocking treatment, obtained in the step (6), was submerged in the solution, and was subjected to a hybridization treatment at a room temperature (25°C) for 3 hours. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (8) Results

As a result of analyzing the results of fluorescence scanner evaluation in the step (7), the fluorescence intensity was 15750 at 532 nm on the spot of the DNA probe of Formula (1) completely matching with the labeled single-stranded DNA probe. In addition, the fluorescence intensity of an area other than the spot area of the DNA probe was observed and found to be 108. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### [Example 16]

### (1) Synthesis of probe

The single-stranded DNA probe was distributed and subjected to dry-up drying just in the same way as Example 15 described above.

(2) Material having functional group of site capable of being bound to probe and functional group of site capable of being bound on substrate (probe binding material)

For preparation of a material having a functional group of a site capable of being bound to a probe and a functional group of a site capable of being bound on a substrate, a silane coupling agent (trade name: KBE-9007 manufactured by The Shin-Etsu Chemical Co., Ltd.) containing a silane compound (3-isocyanatepropyltriethoxy silane) having an isocyanate group was used. The isocyanate silane coupling agent was used as a solution of probe binding material in the following experiment.

### (3) Preparation of probe medium

20 µl of isocyanate silane coupling agent as a probe binding material was dropped and mixed in the micro tube containing the distributed single-stranded DNA probe of the Formula (1) and subjected to dry-up drying. Then, 50 µl of pure water was dropped and mixed therein. After mixing sufficiently for 5 minutes, the mixture was left to stand for 30 minutes.

Then, 500 µl of ethylene glycol and 500 µl of ethylene alcohol as high boiling solvents were dropped in the above micro tube, and were thereafter mixed for 1 minute.

Then, 100 µl of aqueous solution of 0.5 wt% of polyvinyl alcohol (PVA) as a water-soluble polymer material was dropped therein and thereafter mixed for 1 minute. Finally, pure water was dropped therein so that the total amount of probe material was 2000 µl, and was mixed for 5 minutes. Thereafter, the mixture was left to stand for 60 minutes to prepare the probe medium.

### (4) Cleaning of substrate

A substrate was cleaned just in the same way as Example 15 described above to prepare a substrate.

### (5) Spotting of probe medium

The probe medium prepared in the step (3) was loaded in an ink tank for a bubble jet printer (trade name: BJF850; manufactured by Canon Inc.) to be installed in a bubble jet head. The bubble jet printer (trade name: BJF850; manufactured by Canon Inc.) used here had been subjected to a modification so that printing on a plane plate could be done. Then, the glass substrate prepared in the step (4) was installed in this printer, and the probe medium was spotted on the glass substrate. Here, the distance between the liquid discharge surface of the bubble jet head and the liquid retention surface of the glass substrate was in the range of from 1.2 to 1.5 mm. After the spotting process was completed, the glass substrate was observed by a microscope, and as a result it was found that a matrix shaped spot array was formed on the surface of the glass substrate. In addition, the contact angles of pure water were measured on outer peripheries of spot formation sites. As a result, the angle of contact was about 5° in every site of the plate. From this result, it could be found that contaminations were not caused by the probe medium and the like except for spot formation sites. The glass plate after undergoing the spotting process was left to stand in a desiccator. A substrate with an immobilized probe was fabricated in this way.

### (6) Blocking treatment

The substrate with the immobilized probe fabricated in the step (5) was sufficiently washed with a 1M NaCl/50 mM phosphate buffer solution (pH 7.0) to wet the surface of the substrate with the immobilized probe, thereby making it possible to carry out a blocking treatment uniformly. Then, the glass substrate was submerged in an aqueous solution of 2% bovine serum albumin, and was then left to stand for 2 hours to carry out a blocking treatment.

### (7) Hybridization treatment

A single-stranded DNA having a base sequence complementary to the single-stranded DNA probe of Formula (1) described in the step (1) was synthesized in a DNA automatic synthesizer to obtain a single-stranded DNA probe labeled with rhodamine bound to the 5' terminal. This labeled singles-stranded DNA is dissolved in the 1M NaCl/50 mM phosphate buffer solution (pH 7.0) to obtain the 1 µM solution of the labeled DNA, and the substrate with the immobilized probe subjected to the blocking treatment, obtained in the step (6), was submerged in the solution, and was subjected to a hybridization treatment at a room temperature (25°C) for 3 hours. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (8) Results

As a result of analyzing the results of fluorescence scanner evaluation in the step (7), the fluorescence intensity was 15750 at 532 nm on the spot of the DNA probe of Formula (1) completely matching with the labeled single-stranded DNA probe. In addition, the fluorescence intensity of an area other than the spot area of the DNA probe was observed and found to be 108. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### [Comparison Example 1]

### (1) Synthesis of probe

A single-stranded DNA probe was used as a probe capable of being specifically bound to a target material. A single-stranded nucleic acid of SEQ ID NO:10 was synthesized using a DNA automatic synthesizer, and a mercapto (SH) group was introduced into the terminal of the single-stranded nucleic acid by using Thiol-Modifier (manufactured by GlenResearch Co., Ltd.) during synthesis by the DNA automatic synthesizer. Subsequently, normal deprotection was performed, and the DNA was collected and purified by high performance liquid chromatography to obtain an oligomer of Formula (10) for use in the following experiment.

5'HS-(CH₂)₆-O-PO₂-O-ACTGGCCGTCGTTTTACA3' (10)

### (2) Preparation of probe medium

A solution of single-stranded DNA probe of Formula (10) was prepared just in the same way as Example 1 described above (accurate concentration was calculated from absorption intensity). The prepared probe solution was used in the following experiment.

100 µl of probe solution prepared as described above was prepared, and 900 µl of solution containing 7.5 wt% of glycerin, 7.5 wt% of urine, and 7.5 wt% of thiodiglycol was dropped therein. After mixing for 5 minutes, the mixture was left to stand for 30 minutes to prepare a probe medium.

### (3) Cleaning of substrate

A substrate was cleaned just in the same way as Example 1 described above to prepare a glass substrate.

### (4) Probe-immobilization treatment

An aqueous solution of 1 wt% of silane coupling agent (trade name: KBM-603 manufactured by The Shin-Etsu Chemical Co., Ltd.) containing a silane compound (N-β (aminoethyl) γ-aminopropyltrimethoxy silane) having an amino group was stirred at a room temperature for 30 minutes to prepare an aqueous solution of silane coupling agent. Then, the glass substrate cleaned in the step (3) was submerged in the aqueous solution at a room temperature (25°C) for 20 minutes, and was thereafter rinsed sufficiently with pure water to remove excessive solution of silane coupling agent. Nitrogen gas was blown over the both faces of the rinsed substrate for drying. The substrate was then baked for 1 hour in an oven heated at 120°C to complete the silane coupling treatment. Then, 2.7 mg of N-(6-maleimidocaproyloxy) sulfosuccinimide (trade name: EMCS manufactured by Dojindo Laboratories, ltd.) was weighed, and was dissolved in a solution of dimethyl sulfoxide (DMSO)/ethanol (1:1) so that the final concentration was 0.3 mg/ml, whereby an EMCS solution was prepared. The substrate subjected to the silane coupling treatment was submerged in this EMCS solution at a room temperature for 30 minutes. The substrate taken out from the EMCS solution was washed in succession with a mixed solvent of DMSO and ethanol and ethanol, and was thereafter dried under the presence of nitrogen gas. The probe-immobilization treatment was completed in this way. For checking the effect of the probe-immobilization treatment, the contact angle of pure water was measured. As a result, tho contact angle on the substrate was 25 to 30°. From this result, it could be found that the treatment of immobilizing the probe on the substrate had been accomplished appropriately.

### (5) Spotting of probe medium

The probe medium prepared in the step (2) was spotted just in the same way as Example 1 described above to fabricate a substrate with an immobilized probe. After the spotting process was completed, the glass substrate was observed by a microscope, and as a result it was found that a matrix shaped spot array was formed on the surface of the glass substrate. In addition, for checking the effect of the probe-immobilization treatment, the contact angle of pure water was measured. As a result, the contact angle on the substrate was 25 to 30°. From this result, it could be found that contaminations were not caused by the probe medium and the like except for spot formation sites. The glass substrate after undergoing the spotting process was submerged in a container filled with a 1 M NaCl/50 mM phosphate buffer solution (pII 7.0), and was put in a refrigerator together with the container to be stored at a low temperature (4°C). The substrate with the immobilized probe was fabricated in this way.

### (6) Blocking treatment

A blocking treatment was carried out just in the same way as Example 1 described above.

### (7) Hybridization treatment

A single-stranded DNA having a base sequence complementary to the single-stranded DNA probe of Formula (10) described in the step (1) was synthesized in a DNA automatic synthesizer to obtain a single-stranded DNA probe labeled with rhodamine bound to the 5' terminal. This labeled singles-stranded DNA is dissolved in the 1M NaCl/50 mM phosphate buffer solution (pH 7.0) to obtain the 1 µM solution of the labeled DNA, and the substrate with the immobilized probe subjected to the blocking treatment, obtained in the step (6), was submerged in the solution, and was subjected to a hybridization treatment at a room temperature (25°C) for 3 hours. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (8) Results

As a result of analyzing the results of fluorescence scanner evaluation in the step (7), the fluorescence intensity was 12790 at 532 nm on the spot of the DNA probe of Formula (10) completely matching with the labeled single-stranded DNA probe. In addition, the fluorescence intensity of an area other than the spot area of the DNA probe was observed and found to be 792. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### (Example 17)

### (1) Synthesis of probe

A single-stranded DNA probe of Formula (5) was synthesized as a probe capable of being specifically bound to a target material.

### (2) Water-soluble polymer material

Polyvinyl alcohol (PVA) was selected as a water-soluble polymer material. 5 g of polyvinyl alcohol (PVA) (trade name: Poval PVA-117 manufactured by Kuraray Co., Ltd.) was weighed and put in a beaker. This polyvinyl alcohol has a polymerization degree of 1700 and a saponification degree of 98.0 to 99.0 mol%. 495 g of pure water was added to dissolve the polyvinyl alcohol, thereby preparing 1.0 wt% of aqueous polyvinyl alcohol solution. At the time of dissolving the polyvinyl alcohol, the solution was heated at 80°C in a hot bus while it was stirred for 60 minutes so that the polyvinyl alcohol was completely dissolved. After it was checked that no insoluble matter existed, the solution was subjected to filtering to prevent clogging of a nozzle in spotting. A 0.22 µm membrane filter was used for the filtering. In this way, the aqueous PVA solution was prepared.

### (3) Preparation of probe medium

The single-stranded DNA probe of Formula (5) was dissolved in a TE solution (10 mM Tris-HCl (pH8)/1 mM, aqueous solution of ethylenediaminetetraacetic acid (EDTA)) so that the final concentration was about 40 µmol/l, thereby preparing a solution of single-stranded DNA probe (accurate concentration was calculated from absorption intensity).

Then, as a probe immobilizing material, an aqueous solution of 500 µmol/l of silane coupling agent (trade name: KBM-603 manufactured by The Shin-Etsu Chemical Co., Ltd.) containing a silane compound (N-β (aminoethyl) γ-aminopropyltrimethoxy silane) having an amino group was stirred at a room temperature for 30 minutes to prepare an aqueous solution of amino silane. Then, an aqueous solution of 500 µmol/l of bivalent reagent (N-(6-maleimidocaproyloxy) sulfosuccinimide, sodium salt (trade name: sulfo-EMCS; manufactured by Dojindo Corporate, ltd.)) having a maleimide group and a hydroxysuccinimide ester group was stirred at a room temperature for 5 minutes to prepare an aqueous solution of EMCS. The amino silane solution and the EMCS solution were mixed in equal volumes, and were stirred for 5 minutes to obtain a solution of probe immobilizing material. The prepared solution of probe immobilizing material was used in the following experiment.

100 µl of probe solution prepared as described above was prepared, and 100 µl of solution of probe binding material was dropped therein, and was mixed for 5 minutes. After the mixture was left to stand for 10 minutes, 700 µl of pure water and 100 µl of PVA solution as a water-soluble polymer material prepared as described above were dropped therein, and were mixed for 5 minutes. After mixing, the mixture was left to stand for 30 minutes to prepare a probe medium.

### (4) Cleaning of substrate

A glass substrate of an inch square was placed in a cassette, and was submerged for 10 minutes in 1 mol/l of sodium hydroxide solution heated to 60°C in advance. Subsequently, they were sufficiently rinsed in flowing pure water to wash away sodium hydroxide stuck to the glass substrate and cassette. After rinsing sufficiently, the glass substrate together with the cassette was submerged in pure water, and was subjected to ultra sonic cleaning for 10 minutes. After the ultra sonic cleaning, they were sufficiently rinsed in flowing pure water to wash away particles stuck to the glass substrate and the cassette. The rinsed glass substrate was dried one by one with a nitrogen gas blow.

### (5) Spotting of probe medium

The probe medium prepared in the step (3) was loaded in an ink tank for a bubble jet printer (trade name: BJF850; manufactured by Canon Inc.) to be installed in a bubble jet head. The bubble jet printer (trade name: BJF850; manufactured by Canon Inc.) used here had been subjected to a modification so that printing on a plane plate could be done. Then, the glass substrate prepared in the step (4) was installed in this printer, and the probe medium was spotted on the glass substrate. Here, the distance between the liquid discharge surface of the bubble jet head and the liquid retention surface of the glass substrate was in the range of from 1.2 to 1.5 mm. After the spotting process was completed, the glass substrate was observed by a microscope, and as a result it was found that a matrix shaped spot array was formed on the surface of the glass substrate. The glass plate after undergoing the spotting process was left to stand in a desiccator. A substrate with an immobilized probe was fabricated in this way.

### (6) Storage

The substrate with the immobilized probe fabricated in the step (5) was stored in a desiccator for 7 days. An adjustment was made so that the substrate with the immobilized probe was kept at a humidity of 35% or lower and a temperature of 25°C during storage.

### (7) Blocking treatment

The substrate with the immobilized probe stored in the step (6) was washed with a 1M NaCl/50 mM phosphate buffer solution (pH 7.0) to wet the surface of the substrate with the immobilized probe, thereby making it possible to carry out a blocking treatment uniformly. Then, the glass substrate was submerged in an aqueous solution of 2% bovine serum albumin, and was then left to stand for 2 hours to carry out a blocking treatment.

### (8) Hybridization treatment

A single-stranded DNA having a base sequence complementary to the single-stranded DNA probe of Formula (5) described in the step (1) was synthesized in a DNA automatic synthesizer to obtain a single-stranded DNA probe labeled with rhodamine bound to the 5' terminal. This labeled singles-stranded DNA is dissolved in the 1M NaCl/50 mM phosphate buffer solution (pH 7.0) to obtain the 1 µM solution of the labeled DNA, and the substrate with the immobilized probe subjected to the blocking treatment, obtained in the step (7), was submerged in the solution, and was subjected to a hybridization treatment at a room temperature (25°C) for 3 hours. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by AxonInstruments, Inc.). For evaluating the fluorescence intensity, the laser power was set at 100%, and the PMT was set at 400 V.

### (9) Results

As a result of analyzing the results of fluorescence scanner evaluation in the step (8), the fluorescence intensity was 15830 at 532 nm on the spot of the DNA probe of Formula (1) completely matching with the labeled single-stranded DNA probe. In addition, the fluorescence intensity of an area other than the spot area of the DNA probe was observed and found to be 611. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### (Example 18)

### (1) Synthesis of probe

A single-stranded DNA probe was prepared just in the same way as Example 17 described above for use in the following experiment.

### (2) Water-soluble polymer material

An aqueous solution of water-soluble polyvinyl alcohol was prepared in the same way as Example 17 described above. As a water-soluble polymer material, polyvinyl alcohol (PVA) (trade name: Poval PVA-110 manufactured by Kuraray Co., Ltd.) was used. This polyvinyl alcohol has a polymerization degree of 1000 and a saponification degree of 98.0 to 99.0 mol%.

### (3) Preparation of probe medium

A probe solution was prepared in the same way as Example 17 described above.

100 µl of probe solution prepared as described above was prepared, and 800 µl of solution containing 7.5 wt% of glycerin, 7.5 wt% of urine, and 7.5 wt% of thiodiglycol was dropped therein. 100 µl of the aqueous solution of water-soluble polymer material was dropped, and was thereafter mixed for 5 minutes. After mixing, the mixture was left to stand for 30 minutes to obtain a probe medium.

### (4) Cleaning of substrate

A substrate was cleaned just in the same way as Example 17 described above to prepare a glass substrate.

### (5) Probe-immobilization treatment

An aqueous solution of 1 wt% of silane coupling agent (trade name: KBM-603 manufactured by The Shin-Etsu Chemical Co., Ltd.) containing a silane compound (N-β (aminoethyl) γ-aminopropyltrimethoxy silane) having an amino group was stirred at a room temperature for 30 minutes to prepare an aqueous solution of silane coupling agent. Then, the glass substrate cleaned in the step (4) was submerged in the aqueous solution at a room temperature (25°C) for 20 minutes, and was thereafter rinsed sufficiently with pure water to remove excessive solution of silane coupling agent. Nitrogen gas was blown over the both faces of the rinsed substrate for drying. The substrate was then baked for 1 hour in an oven heated at 120°C to complete the silane coupling treatment. Then, 2.7 mg of N-(6-maleimidocaproyloxy) sulfosuccinimide (trade name: EMCS manufactured by Dojindo Laboratories, ltd.) was weighed, and was dissolved in a solution of dimethyl sulfoxide (DMSO)/ethanol (1:1) so that the final concentration was 0.3 mg/ml, whereby an EMCS solution was prepared. The substrate subjected to the silane coupling treatment was submerged in this EMCS solution at a room temperature for 30 minutes. The substrate taken out from the EMCS solution was washed in succession with a mixed solvent of DMSO and ethanol and ethanol, and was thereafter dried under the presence of nitrogen gas. The probe immobilizing treatment was completed in this way.

### (6) Spotting of probe medium

The probe medium prepared in the step (3) was spotted just in the same way as Example 17 described above to fabricate a substrate with an immobilized probe. After the spotting process was completed, the glass substrate was observed by a microscope, and as a result it was found that a matrix shaped spot array was formed on the surface of the glass substrate. The glass plate after undergoing the spotting process was left to stand in a desiccator. A substrate with an immobilized probe was fabricated in this way.

### (7) Storage

The substrate with the immobilized probe fabricated in the step (6) was stored in a desiccator for 7 days. An adjustment was made so that the substrate with the immobilized probe was kept at a humidity of 35% or lower and a temperature of 25°C during storage.

### (8) Blocking treatment

A blocking treatment was carried out just in the same way as Example 17 described above.

### (9) Hybridization treatment

A hybridization treatment was carried out just in the same way as Example 17 described above. Thereafter, the probe array was washed with the 1 M NaCl/50 mM phosphate buffer solution (pH 7.0) to wash away single-stranded DNA probe not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (10) Results

As a result of analyzing the results of fluorescence scanner evaluation in the step (9), the fluorescence intensity was 11520 at 532 nm on the spot of the DNA probe of Formula (5) completely matching with the labeled single-stranded DNA probe. In addition, the fluorescence intensity of an area other than the spot area of the DNA probe was observed and found to be 659. When the spot of each DNA probe was.observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### (Example 19)

### (1) Synthesis of probe

Single-stranded DNA probes of Formulae (5) to (8) used in Example 10 were synthesized for use in the following experiment.

### (2) Water-soluble polymer material

A PVA solution was prepared just in the same way as Example 17.

### (3) Preparation of probe medium

A solution of single-stranded DNA probe was prepared just in the same way as Example 17 described above.

The single-stranded DNAs of SEQ ID NO:5 to 8 were used to prepare four types of probe medium in the same way as described in the step (3) of Example 17 described above (accurate concentration was calculated from absorption intensity).

### (4) Cleaning of substrate

A substrate was cleaned just in the same way as Example 17 described above to prepare a glass substrate.

### (5) Spotting of probe medium

The four probe mediums prepared in the step (3) were loaded in four ink tanks for a bubble jet printer (trade name: BJF850; manufactured by Canon Inc.), respectively, and each of the ink tanks was installed in a bubble jet head. The bubble jet printer (trade name: BJF850; manufactured by Canon Inc.) used here had been subjected to a modification so that printing on a plane plate could be done. Then the glass substrate prepared in the step (4) was installed in this printer, and the probe medium was spotted on the glass substrate. Here, the distance between the liquid discharge surface of the bubble jet head and the liquid retention surface of the glass substrate was in the range of from 1.2 to 1.5 mm. After the spotting process was completed, the glass substrate was observed by a microscope, and as a result it was found that a matrix shaped spot array was formed on the surface of the glass substrate. The glass plate after undergoing the spotting process was left to stand in a desiccator. A substrate with an immobilized probe with four types of probes immobilized thereon was fabricated in this way.

### (6) Storage

The substrate with the immobilized probe fabricated in the step (5) was stored in a desiccator for 7 days. An adjustment was made so that the substrate with the immobilized probe was kept at a humidity of 35% or lower and a temperature of 25°C during storage.

### (7) Blocking treatment

The surface of the substrate was washed with a 1 M NaCl/50 mM phosphate buffer solution (pH 7.0), thereby making it possible to carry out a blocking treatment uniformly, without conducting the blocking treatment carried out in Example 17 described above.

### (8) Hybridization treatment

A hybridization treatment was carried out just in the same way as Example 17 described above. Thereafter, the probe array was washed with a 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (9) Results

As a result of analyzing the results of fluorescence scanner evaluation in the step (7), the fluorescence intensity was 15120 at 532 nm on the spot of the DNA probe of Formula (5) completely matching with the labeled single-stranded DNA probe. In contrast to this, for the spot of the DNA probe of Formula (6) having a one-base mismatch sequence, the fluorescence intensity was 9030. Also, for the spot of the DNA probe of Formula (7) having a three-base mismatch sequence, the fluorescence intensity was 3423, which was less than half of the fluorescence intensity of the completely matching DNA probe, and for the DNA probe of Formula (8) having a six-base mismatch, little fluorescence spot could be recognized where the fluorescence intensity at the site corresponding to the spot was 973. In addition, the fluorescence intensity of an area other than the spot area of the DNA probe was observed and found to be 653. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm. From the above, a fully complementary single stranded DNA could be detected specifically on the DNA array substrate.

### (Example 20)

### (1) Synthesis of probe

The single-stranded DNA probe of Formula (1) used in Example 1 was used as a probe capable of being specifically bound to a target material. A DNA automatic synthesizer was used to synthesis a single-stranded DNA probe of Formula (5). Furthermore, an octadecanomeric oligomer with an amino group bound to the 5' terminal hydroxyl group of the single-stranded DNA of Formula (1) via a phosphate group and hexamethylene was prepared for use in the following experiment.

### (2) Water-soluble polymer material

Polyvinyl alcohol (PVA) was selected as a water-soluble polymer material. 5 g of polyvinyl alcohol (PVA) (trade name: Poval PVA-217E manufactured by Kuraray Co., Ltd.) was weighed and put in a beaker. This polyvinyl alcohol has a polymerization degree of 1700 and a saponification degree of 87.0 to 89.0 mol%. 495 g of pure water was added to dissolve the polyvinyl alcohol, thereby preparing 1.0 wt% of aqueous polyvinyl alcohol solution. At the time of dissolving the polyvinyl alcohol, the solution was heated at 80°C in a hot bus while it was stirred for 60 minutes so that the polyvinyl alcohol was completely dissolved. After it was checked that no insoluble matter existed, the solution was subjected to filtering to prevent clogging of a nozzle in spotting. A 0.22 µm membrane filter was used for the filtering. In this way, the aqueous PVA solution was prepared.

### (3) Preparation of probe medium

The single-stranded DNA probe of Formula (5) was dissolved in a TE solution (10 mM Tris-HCL (pH8)/1 mM, aqueous solution of ethylenediaminetetraacetic acid (EDTA)) so that the final concentration was about 40 µmol/l, thereby preparing a solution of single-stranded DNA probe (accurate concentration was calculated from absorption intensity).

Then, as a probe immobilizing material, an aqueous solution of 500 µmol/l of silane coupling agent (trade name: KBM-403 manufactured by The Shin-Etsu Chemical Co., Ltd.) containing a silane compound (γ-glycidoxypropyltrimethoxy silane) having an epoxy group was stirred at a room temperature for 30 minutes to obtain a solution of probe immobilizing material.

100 µl of probe solution prepared as described above was prepared, and 100 µl of solution of probe binding material was dropped therein, and was mixed for 5 minutes. After the mixture was left to stand for 10 minutes, 700 µl of pure water and 100 µl of PVA solution as a water-soluble polymer material prepared as described above were dropped therein, and were mixed for 5 minutes. After mixing, the mixture was left to stand for 30 minutes to prepare a probe medium.

### (4) Cleaning of substrate

A substrate was cleaned just in the same way as Example 17 described above to prepare a glass substrate.

### (5) Spotting of probe medium

The probe medium was spotted on the substrate of the probe medium in the same way as Example 17 described above to prepare a glass substrate with an immobilized probe.

### (6) Storage

The substrate with the immobilized probe fabricated in the step (5) was stored in a desiccator for 7 days. An adjustment was made so that the substrate with the immobilized probe was kept at a humidity of 35% or lower and a temperature of 25°C during storage.

### (7) Blocking treatment

The substrate with the immobilized probe stored in the step (6) was washed with a 1M NaCl/50 mM phosphate buffer solution (pH 7.0) to wet the surface of the substrate with the immobilized probe, thereby making it possible to carry out a blocking treatment uniformly. Then, the glass substrate was submerged in an aqueous solution of 2% bovine serum albumin, and was then left to stand for 2 hours to carry out a blocking treatment.

### (8) Hybridization treatment

A single-stranded DNA having a base sequence complementary to the single-stranded DNA probe of Formula (1) described in the step (1) was synthesized in a DNA automatic synthesizer to obtain a single-stranded DNA probe labeled with rhodamine bound to the 5' terminal. This labeled singles-stranded DNA is dissolved in the 1M NaCl/50 mM phosphate buffer solution (pH 7.0) to obtain the 1µM solution of the labeled DNA, and the substrate with the immobilized probe subjected to the blocking treatment, obtained in the step (7), was submerged in the solution, and was subjected to a hybridization treatment at a room temperature (25°C) for 3 hours. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (9) Results

As a result of analyzing the results of fluorescence scanner evaluation in the step (8), the fluorescence intensity was 17110 at 532 nm on the spot of the DNA probe of Formula (1) completely matching with the labeled single-stranded DNA probe. In addition, the fluorescence intensity of an area other than the spot area of the DNA probe was observed and found to be 731. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### (Example 21)

### (1) Synthesis of probe

A single-stranded DNA probe of Formula (1) was prepared just in the same way as Example 17 described above for use in the following experiment.

### (2) Water-soluble polymer material

An aqueous PVA solution was prepared just in the same way as Example 17 described above.

### (3) High boiling solvent

Propylene glycol (1,2-propanediol manufactured by Kishida Chemical Co., Ltd) was selected as a high boiling solvent. 5.2 g of propylene glycol was weighed, and 4 g of isopropyl alcohol (2-propanol manufactured by Kishida Chemical Co., Ltd) was weighed and mixed with the propylene glycol. The isopropyl alcohol was mixed in consideration of extractability and mixability during preparation of a probe medium. A high boiling solvent liquid was prepared in this way.

### (4) Preparation of probe medium

The above single-stranded DNA probe of Formula (1) was distributed among micro tubes so that one micro tube contained 17.53 nmol of single-stranded DNA probe, and was dried to prepare micro tubes each containing a single-stranded DNA probe.

One micro tube containing the single-stranded DNA probe distributed as described above was prepared, and 50 µl of pure water was dropped therein, and was thereafter stirred for 3 minutes to completely dissolve the DNA. 20 µl of silane coupling agent (trade name: KBM-403 manufactured by The Shin-Etsu Chemical Co., Ltd.) containing a silane compound (y-glycidoxypropyltrimethoxy silane) having an epoxy group was dropped therein as a probe immobilizing material, and was mixed for 10 minutes. The mixture was left to stand for 30 minutes, and thereafter 100 µl of aqueous PVA solution as the water-soluble polymer material and 1400 µl of high boiling solvent liquid were dropped therein, and were mixed for 5 minutes. Finally, 430 µl of pure water was dropped therein and mixed for 5 minutes, and was thereafter stirred for 30 minutes. A probe medium was prepared in this way.

### (5) Cleaning of substrate

A substrate was cleaned just in the same way as Example 17 described above to prepare a glass substrate.

### (6) Spotting of probe medium

The probe medium was spotted on the substrate of the probe medium in the same way as Example 17 described above to prepare a glass substrate.

### (7) Storage

The substrate with the immobilized probe fabricated in the step (6) was stored in a desiccator for 7 days. An adjustment was made so that the substrate with the immobilized probe was kept at a humidity of 35% or lower and a temperature of 25°C during storage.

In addition, for checking the dry storability as a substrate with an immobilized probe, the substrate with the immobilized probe was dried on a hotplate at 80°C for 5 minutes, and was thereafter stored similarly in a desiccator for 7 days.

### (8) Hybridization treatment

A single-stranded DNA having a base sequence complementary to the single-stranded DNA probe of Formula (1) described in the step (1) was synthesized in a DNA automatic synthesizer to obtain a single-stranded DNA probe labeled with rhodamine bound to the 5' terminal. This labeled single-stranded DNA was dissolved in the 1M NaCl/50 mM phosphate buffer solution (pH 7-0) so that the final concentration was 1 µM, and the substrate with the immobilized probe was submerged in the solution, and was subjected to a hybridization treatment in an incubator (45°C) for 2 hours. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (9) Results

As a result of analyzing the results of fluorescence scanner evaluation in the step (8), the fluorescence intensity was 12970 at 532 nm on the spot of the DNA probe of Formula (1) completely matching with the labeled single-stranded DNA probe, for the substrate with the immobilized probe stored in the desiccator for 7 days. In addition, the fluorescence intensity of an area other than the spot area of the DNA probe was observed and found to be 131. In contrast to this, the fluorescence intensity was 13540 at 532 nm on the spot of the DNA probe of Formula (5) completely matching with the labeled single-stranded DNA probe, for the substrate with the immobilized probe dried on the hotplate at 80°C for 5 minutes. In addition, the fluorescence intensity of an area other than the spot area of the DNA probe was observed and found to be 96. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### (Example 22)

### (1) Synthesis of probe

A single-stranded DNA probe of Formula (1) was prepared just in the same way as Example 20 described above for use in the following experiment.

### (2) Water-soluble polymer material

An aqueous PVA solution was prepared just in the same way as Example 17 described above.

### (3) High boiling solvent

Propylene glycol (1,2-propanediol manufactured by Kishida Chemical Co., Ltd) was selected as a high boiling solvent. 5.6 g of propylene glycol was weighed, and 7.9 g of isopropyl alcohol (2-propanol manufactured by Kishida Chemical Co., Ltd) was weighed and mixed with the propylene glycol. The isopropyl alcohol was mixed in consideration of extractability and mixability during preparation of a probe medium. A high boiling solvent liquid was prepared in this way.

### (4) Preparation of probe medium

The above single-stranded DNA probe of Formula (1) was distributed among micro tubes so that one micro tube contained 17.53 nmol of single-stranded DNA probe, and was dried to prepare micro tubes each containing a single-stranded DNA probe.

One micro tube containing the single-stranded DNA probe distributed as described above was prepared, and 50 µl of pure water was dropped therein, and was thereafter stirred for 3 minutes to completely dissolve the DNA. Twenty µl of silane coupling agent (trade name: KBE-9007 manufactured by The Shin-Etsu Chemical Co., Ltd.) containing a silane compound (y-isocyanatepropyltriethoxy silane) having an isocyanate group was dropped therein as a probe immobilizing material, and was mixed for 10 minutes. The mixture was' left to stand for 30 minutes, and thereafter 100 µl of aqueous PVA solution as the water-soluble polymer material and 1400 µl of high boiling solvent liquid were dropped therein, and were mixed for 5 minutes. Finally, 430 µl of pure water was dropped therein and mixed for 5 minutes, and was thereafter stirred for 30 minutes. A probe medium was prepared in this way.

### (5) Cleaning of substrate

A substrate was cleaned just in the same way as Example 17 described above to prepare a glass substrate.

### (6) Spotting of probe medium

The probe medium was spotted on the substrate of the probe medium in the same way as Example 17 described above to prepare a glass substrate.

### (7) Storage

The substrate with the immobilized probe fabricated in the step (5) was stored in a desiccator for 7 days. An adjustment was made so that the substrate with the immobilized probe was kept at a humidity of 35% or lower and a temperature of 25°C during storage.

In addition, for checking the dry storability as a substrate with an immobilized probe, the substrate with the immobilized probe was dried on a hotplate at 80°C for 5 minutes, and was thereafter stored similarly in a desiccator for 7 days.

### (8) Hybridization treatment

A single-stranded DNA having a base sequence complementary to the single-stranded DNA probe of Formula (1) described in the step (1) was synthesized in a DNA automatic synthesizer to obtain a single-stranded DNA probe labeled with rhodamine bound to the 5' terminal. This labeled singles-stranded DNA is dissolved in the 1M NaCl/50 mM phosphate buffer solution (pH 7.0) to obtain the 1µM solution of the labeled DNA, and the substrate with the immobilized probe was submerged in the solution, and was subjected to a hybridization treatment in an incubator (45°C) for 2 hours. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (9) Results

As a result of analyzing the results of fluorescence scanner evaluation in the step (8), the fluorescence intensity was 17790 at 532 nm on the spot of the DNA probe of Formula (1) completely matching with the labeled single-stranded DNA probe, for the substrate with the immobilized probe stored in the desiccator for 7 days. In addition, the fluorescence intensity of an area other than the spot area of the DNA probe was observed and found to be 89. In contrast to this, the fluorescence intensity was 18340 at 532 nm on the spot of the DNA probe of Formula (5) completely matching with the labeled single-stranded DNA probe, for the substrate with the immobilized probe dried on the hotplate at 80°C for 5 minutes. In addition, the fluorescence intensity of an area other than the spot area of the DNA probe was observed and found to be 81. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### (Comparison Example 2)

### (1) Synthesis of probe

A single-stranded DNA probe of Formula (5) was prepared just in the same way as Example 17 describe above for use in the following experiment.

### (2) Water-soluble polymer material

For checking the effect by a water-soluble polymer material, a substrate with an immobilized probe was fabricated without using the water-soluble polymer material.

### (3) Preparation of probe medium

A solution of single-stranded DNA probe of Formula (5) was prepared just in the same way as Example 17 described above (accurate concentration was calculated from absorption intensity). Then, a solution of probe immobilizing material constituted by amino silane and a bivalent reagent was prepared in the same way as Example 17. The prepared solution of probe immobilizing material was used in the following experiment.

100 µl of probe solution prepared as described above was prepared, and 100 µl of solution of probe binding material was dropped therein, and was mixed for 5 minutes. After the mixture was left to stand for 10 minutes, 800 µl of pure water was dropped therein, and was mixed for 5 minutes. After mixing, the mixture was left to stand for 30 minutes to prepare a probe medium.

### (4) Cleaning of substrate

A substrate was cleaned just in the same way as Example 17 described above to prepare a glass substrate.

### (5) Spotting of probe medium

The probe medium was spotted on the substrate of the probe medium in the same way as Example 17 described above to prepare a glass substrate with the immobilized probe.

### (6) Storage

The substrate with the immobilized probe fabricated in the step (5) was stored in a desiccator for 7 days. An adjustment was made so that the substrate with the immobilized probe was kept at a humidity of 35% or lower and a temperature of 25°C during storage.

### (7) Blocking treatment

The substrate with the immobilized probe stored in the step (6) was washed with a 1M NaCl/50 mM phosphate buffer solution (pH 7.0) to wet the surface of the substrate with the immobilized probe, thereby making it possible to carry out a blocking treatment uniformly. Then, the glass substrate was submerged in an aqueous solution of 2% bovine serum albumin, and was then left to stand for 2 hours to carry out a blocking treatment.

### (8) Hybridization treatment

A single-stranded DNA having a base sequence complementary to the single-stranded DNA probe of Formula (5) described in the step (1) was synthesized in a DNA automatic synthesizer to obtain a single-stranded DNA probe labeled with rhodamine bound to the 5' terminal. This labeled singles-stranded DNA is dissolved in the 1M NaCl/50 mM phosphate buffer solution (pH 7.0) to obtain the 1 µM solution of the labeled DNA, and the substrate with the immobilized probe subjected to the blocking treatment, obtained in the step (7), was submerged in the solution, and was subjected to a hybridization treatment at a room temperature (25°C) for 3 hours. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (9) Results

As a result of analyzing the results of fluorescence scanner evaluation in the step (8), the fluorescence intensity was 1427 at 532 nm on the spot of the DNA probe of Formula (5) completely matching with the labeled single-stranded DNA probe. In addition, the fluorescence intensity of an area other than the spot area of the DNA probe was observed and found to be 697. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm. In this way, when a probe medium containing no water-soluble polymer material was used, the fluorescence intensity at the spot site in complete matching was insufficient, thus making it impossible to carry out detection effectively.

### (Comparison Example 3)

### (1) Synthesis of probe

A single-stranded DNA probe of Formula (5) was prepared just in the same way as Example 1 describe above for use in the following experiment.

### (2) Water-soluble polymer material

For checking the effect by a water-soluble polymer material, a substrate with an immobilized probe was fabricated without using the water-soluble polymer material.

### (3) Preparation of probe medium

A solution of single-stranded DNA probe of Formula (5) was prepared just in the same way as Example 17 described above (accurate concentration was calculated from absorption intensity). The prepared probe solution was used in the following experiment.

100 µl of probe solution prepared as described above was prepared, and 900 µl of solution containing 7.5 wt% of glycerin, 7.5 wt% of urine, and 7.5 wt% of ethyleneglycol was dropped therein. After mixing for 5 minutes, the mixture was left to stand for 30 minutes to prepare a probe medium.

### (4) Cleaning of substrate

A substrate was cleaned just in the same way as Example 17 described above to prepare a glass substrate.

### (5) Probe-immobilization treatment

A treatment of immobilizing the probe on the cleaned glass substrate was performed in the same way as Example 18 described above.

### (6) Spotting of probe medium

The probe medium prepared in the step (3) was spotted just in the same way as Example 17 described above to fabricate a substrate with an immobilized probe. After the spotting process was completed, the glass substrate was observed by a microscope, and as a result it was found that a matrix shaped spot array was formed on the surface of the glass substrate. The glass plate after undergoing the spotting process was left to stand in a desiccator. A substrate with an immobilized probe was fabricated in this way.

### (7) Storage

The substrate with the immobilized probe fabricated in the step (6) was stored in a desiccator for 7 days. An adjustment was made so that the substrate with the immobilized probe was kept at a humidity of 35% or lower and a temperature of 25°C during storage.

### (8) Blocking treatment

A blocking treatment was carried out just in the same way as Example 17 described above.

### (9) Hybridization treatment

A hybridization treatment was carried out just in the same way as Example 17 described above. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (10) Results

As a result of analyzing the results of fluorescence scanner evaluation in the step (9), the fluorescence intensity was 1527 at 532 nm on the spot of the DNA probe of Formula (5) completely matching with the labeled single-stranded DNA probe. In addition, the fluorescence intensity of an area other than the spot area of the DNA probe was observed and found to be 792. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

### (Comparison Example 4)

### (1) Synthesis of probe

A single-stranded DNA probe of Formula (1) was prepared just in the same way as Example 20 describe above for use in the following experiment.

### (2) Water-soluble polymer material

For checking the effect by a water-soluble polymer material, a substrate with an immobilized probe was fabricated without using the water-soluble polymer material.

### (3) High boiling solvent

For checking the effect by a high boiling solvent, a substrate with an immobilized probe was fabricated without using the high boiling solvent.

### (4) Preparation of probe medium

A solution of single-stranded DNA probe of Formula (1) was prepared just in the same way as Example 20 described above (accurate concentration was calculated from absorption intensity). Pure water instead of the PVA solution as a water-soluble polymer material of the step (2), and isopropyl alcohol as an alternative for the high boiling solvent of the step (3) were dropped and mixed. The -prepared probe solution was used in the following experiment.

### (5) Cleaning of substrate

A substrate was cleaned in the same way as Example 17 described above to prepare a glass substrate.

### (6) Probe-immobilization treatment

A treatment of immobilizing the probe on the cleaned glass substrate was performed in the same way as Example 17 described above.

### (7) Spotting of probe medium

The probe medium prepared in the step (4) was spotted just in the same way as Example 17 described above to fabricate a substrate with an immobilized probe. After the spotting process was completed, the glass substrate was observed by a microscope, and as a result it was found that a matrix shaped spot array was formed on the surface of the glass substrate. The glass plate after undergoing the spotting process was left to stand in a desiccator. A substrate with an immobilized probe was fabricated in this way.

In addition, some probe immobilizing substrates were treated on a hotplate at 80°C for 5 minutes.

### (8) Storage

The substrate with the immobilized probe fabricated in the step (7) was stored in a desiccator for 7 days. An adjustment was made so that the substrate with the immobilized probe was kept at a humidity of 35% or lower and a temperature of 25°C during storage.

### (9) Hybridization treatment

A hybridization treatment was carried out just in the same way as Example 17 described above. Thereafter, the probe array was washed with the 1 M NaCl/50 mM phosphate buffer solution (pH 7.0) to wash away single-stranded DNA probes not hybridized with the probe nucleic acid. Then, excessive salts were removed with pure water, followed by drying the probe foxing substrate by nitrogen blow. Then, the intensity of fluorescence of the spot on the substrate with the immobilized probe was evaluated using a fluorescence scanner (trade name: GenePix 4000B prepared by Axon Instruments, Inc.). For evaluating the fluorescence intensity, the laser power was set at 100%, and the PMT was set at 400 V.

### (10) Results

As a result of analyzing the results of fluorescence scanner evaluation in the step (9), the fluorescence intensity was 4276 at 532 nm on the spot of the DNA probe of Formula (1) completely matching with the labeled single-stranded DNA probe, for the substrate with the immobilized probe stored in the desiccator for 7 days. In addition, the fluorescence intensity of an area other than the spot area of the DNA probe was observed and found to be 89. In contrast to this, the fluorescence intensity was 1286 at 532 nm on the spot of the DNA probe of Formula (5) completely matching with the labeled single-stranded DNA probe, for the substrate with the immobilized probe dried on the hotplate at 80°C for 5 minutes. In addition, the fluorescence intensity of an area other than the spot area of the DNA probe was observed and found to be 81. When the spot of each DNA probe was observed with fluorescence, each spot appeared to be almost circular, and there was little difference in fluorescence intensity among spots obtained by spotting the same probe medium. In addition, it was observed that the distances between adjacent spots were almost the same, and the spots were placed in a lattice form at intervals of about 200 µm.

## Claims

1. A probe medium comprising:
a single-stranded nucleic acid probe which can specifically bond to a target substance and of which an end is modified with a functional group; and
a silane coupling agent having a functional group which can bond to the probe, and having a silanol group which can bond to a substrate surface, wherein
said medium contains water.

2. Probe medium according to claim 1 wherein the bond is a covalent bond.

3. Probe medium according to claim 1, further comprising a bifunctional agent with which the single-stranded nucleic acid probe and the silane coupling agent are bonded to each other.

4. A process for manufacturing a substrate on which a probe is immobilized comprising the steps of:
providing a substrate; and
providing the substrate with a probe medium according to claim 1.

5. A process for manufacturing a DNA array comprising the steps of:
providing a substrate; and
providing the substrate with a probe medium according to claim 1.

6. A process for detecting a target substance comprising the steps of:
providing the DNA array according to claim 5 wherein the probe is immobilized on the substrate; and
detecting the target substance using the substrate with the immobilized probe.

7. A process for manufacturing a probe medium according to claim 1, comprising the steps of:
providing a single-stranded nucleic acid probe and a silane coupling agent having a functional group which can bond to the end of the single-stranded nucleic acid probe, and having a silanol group;
placing a solution containing the silane coupling agent into a container and;
mixing the probe and the silane coupling agent, whereby the probe and the silane coupling agent are dissolved in an aqueous solution.

8. A DNA array wherein a probe medium as defined in any of claims 1 to 3 is separately placed in a spot form.

## Patentansprüche

1. Sondenmedium, das umfasst:
eine einzelsträngige Nukleinsäuresonde, welche spezifisch an eine Zielsubstanz binden kann, und von welcher ein Ende mit einer funktionellen Gruppe modifiziert ist; und
ein Silankupplungsagens mit einer funktionellen Gruppe, welche an die Sonde binden kann, und eine Silanolgruppe aufweist, welche an eine Substratoberfläche binden kann, wobei
das Medium Wasser enthält.

2. Sondenmedium nach Anspruch 1, wobei die Bindung eine kovalente Bindung ist.

3. Sondenmedium nach Anspruch 1, das ferner ein bifunktionelles Agens, mit welchem die einzelsträngige Nukleinsäuresonde und das Silankupplungsagens aneinander gebunden sind, umfasst.

4. Verfahren zur Herstellung eines Substrats, auf welchem eine Sonde immobilisiert ist, das die Schritte umfasst von:
Bereitstellen eines Substrats; und
Bereitstellen des Substrats mit einem Sondenmedium nach Anspruch 1.

5. Verfahren zur Herstellung einer DNS-Matrix, das die Schritte umfasst von:
Bereitstellen eines Substrats; und
Bereitstellen des Substrats mit einem Sondenmedium nach Anspruch 1.

6. Verfahren zur Detektion einer Zielsubstanz, das die Schritte umfasst von:
Bereitstellen der DNS-Matrix nach Anspruch 5, wobei die Sonde auf dem Substrat immobilisiert ist; und
Detektieren der Zielsubstanz unter Verwendung des Substrats mit der immobilisierten Sonde.

7. Verfahren zur Herstellung eines Sondenmediums nach Anspruch 1, das die Schritte umfasst von:
Bereitstellen einer einzelsträngigen Nukleinsäuresonde und eines Silankupplungsagens mit einer funktionellen Gruppe, welche an das Ende der einzelsträngigen Nukleinsäuresonde binden kann und eine Silanolgruppe aufweist;
Platzieren einer Lösung, die das Silankupplungsagens enthält, in einen Container; und
Mischen der Sonde und des Silankupplungsagens, wodurch die Sonde und das Silankupplungsagens in einer wässrigen Lösung gelöst werden.

8. DNS-Matrix, wobei ein Sondenmedium, wie in einem der Ansprüche 1 bis 3 definiert, in einer Punktform separat platziert ist.

## Revendications

1. Milieu de sonde comprenant :
une sonde d'acide nucléique monocaténaire qui peut se lier spécifiquement à une substance cible et dont une extrémité est modifiée avec un groupe fonctionnel ; et
un agent de couplage du type silane ayant un groupe fonctionnel qui peut se lier à la sonde, et ayant un groupe silanol qui peut se lier à la surface d'un substrat,
ledit milieu contenant de l'eau.

2. Milieu de sonde suivant la revendication 1, dans lequel la liaison est une liaison covalente.

3. Milieu de sonde suivant la revendication 1, comprenant en outre un agent bifonctionnel avec lequel la sonde d'acide nucléique monocaténaire et l'agent de couplage du type silane sont liés l'un à l'autre.

4. Procédé pour la production d'un substrat sur lequel une sonde est immobilisée, comprenant les étapes de :
fourniture d'un substrat ; et
fourniture au substrat d'un milieu de sonde suivant la revendication 1.

5. Procédé pour la production d'une matrice d'ADN, comprenant les étapes de :
fourniture d'un substrat ; et
fourniture au substrat d'un milieu de sonde suivant la revendication 1.

6. Procédé pour la détection d'une substance cible, comprenant les étapes de :
fourniture de la matrice d'ADN suivant la revendication 5, dans lequel la sonde est immobilisée sur le substrat ; et
détection de la substance cible en utilisant le substrat avec la sonde immobilisée.

7. Procédé pour la production d'un milieu de sonde suivant la revendication 1, comprenant les étapes de :
fourniture d'une sonde d'acide nucléique monocaténaire et d'un agent de couplage du type silane ayant un groupe fonctionnel qui peut se lier à l'extrémité de la sonde d'acide nucléique monocaténaire, et ayant un groupe silanol ;
introduction d'une solution contenant l'agent de couplage du type silane dans un récipient ; et
mélange de la sonde et de l'agent de couplage du type silane, la sonde et l'agent de couplage du type silane étant ainsi dissous en solution aqueuse.

8. Matrice d'ADN dans laquelle un milieu de sonde tel que défini dans l'une quelconque des revendications 1 à 3 est placé de manière séparée sous forme de taches.


### SEQUENCE LISTING

<110> Canon Kabushiki Kaisha
<120> probe medium
<130> CF016889WO
<140>
   <141>
<150> JP 2001-394086
   <151> 2001-12-26
<160> 10
<170> PatentIn Ver. 2.1
<210> 1
   <211> 18
   <212> DNA
   <213> Artificial Sequence
<220>
   <223> Description of Artificial Sequence: oligomer for probe
<220>
   <223> Inventor: Okada, Yoshikatsu; Kameyama, Makoto; Iwata, Kenichi
<400> 1
   actggccgtc gttttaca
   18
<210> 2
   <211> 18
   <212> DNA,
   <213> Artificial Sequence
<220>
   <223> Description of Artificial Sequence: oligomer for probe
<400> 2
   actggccgtt gttttaca
   18
<210> 3
   <211> 18
   <212> DNA
   <213> Artificial Sequence
<220>
   <223> Description of Artificial Sequence: olligomer for probe
<400> 3
   actggccgct tttttaca
   18
<210> 4
   <211> 18
   <212> DNA
   <213> Artificial Sequence
<220>
   <223> Description of Artificial Sequence: oligomer for probe
<400> 4
   actggcatct tgtttaca
   18
<210> 5
   <211> 188
   <212> DNA
   <213> Artificial Sequence
<220>
   <223> Description of Artificial Sequence: oligomer for probe
<400> 5
<210> 6
   <211> 18
   <212> DNA
   <213> Artificial Sequence
<220>
   <223> Description of Artificial Sequence: oligomer for probe
<400> 6
   actggccgtt gttttaca
   18
<210> 7
   <211> 18
   <212> DNA
   <213> Artificial Sequence
<220>
   <223> Description of Artificial Sequence: oligomaer for probe
<400> 7
   actggccgct tttttaca
   18
<210> 8
   <211> 18
   <212> DNA
   <213> Artificial Sequence
<220>
   <223> Description of Artificial Sequence: oligomer for probe
<400> 8
   actggcatct tgtttaca
   18
<210> 9
   <211> 18
   <212> DNA
   <213> Artificial Sequence
<220>
   <223> Description of Artificial Sequence: oligomer for probe
<400> 9
   actggccgtc gttttaca
   18
<210> 10
   <211> 18
   <212> DNA
   <213> Artificial Sequence
<220>
   <223> Description of Artificial Sequence: oligomer for probe
<400> 10
   actggccgtc gttttaca
   18